# EUROPEAN PATENT APPLICATION

(11) **EP 2 295 537 A1**
(43) Date of publication of application: **16.03.2011**
(21) Application number: 09746625.4
(22) Date of filing: 13.05.2009
(51) Int. Cl.: C12N 5/00, C12N 5/10, C12Q 1/02, C12N 15/09

(54) **HUMAN HEPATIC STEM CELL, METHOD FOR PREPARATION OF THE SAME, METHOD FOR INDUCTION OF DIFFERENTIATION OF THE SAME, AND METHOD FOR UTILIZATION OF THE SAME**

(30) Priority: 14.05.2008 JP 2008127755
(71) Applicant: PUBLIC UNIVERSITY CORPORATION YOKOHAMA CITY UNIVERSITY, Kanazawa-ku Yokohama-shi Kanagawa 236-0027 (JP)
(72) Inventor: TANIGUCHI, Hideki, Yokohama-shi Kanagawa 236-0004 (JP); ZHENG, Yun-Wen, Yokohama-shi Kanagawa 236-0004 (JP)
(74) Representative: Ford, Hazel
(86) International application number: PCT/JP2009/058932
(87) International publication number: WO 2009/139419

(57) **Abstract**

The present invention provides a human hepatic stem cell which is sorted based on the presence or absence of the expression of at least one marker selected from the group consisting of CD318, CD90, CD66 and CD13. A method of preparing the human hepatic stem cell, a method of inducing differentiation of the same and a method of using the same are also provided.

## Description

### TECHNICAL FIELD

The present invention relates to a human hepatic stem cell, a method of preparing the same, a method of inducing differentiation of the same, and a method of using the same.

### BACKGROUND ART

Conventionally, isolation of stem cells has been performed by centrifugation (Non-Patent Document No. 1). However, this method is prone to contamination of stem cells by non-stem cells. Thus, efficient isolation has been difficult. Under the circumstances, development of a method capable of isolating stem cells alone at higher efficiency has been pursued. According to recent reports, it is believed that isolation methods using monoclonal antibodies to cell surface antigens specific for the stem cells of individual organs are most appropriate, and such methods are frequently used for isolation of hematopoietic stem cells. Although cell surface antigens specific for hepatic stem cells have not been identified yet, the group of the present inventors has reported a method of concentrating hepatic stem cells from mouse livers using the expression of 5 to 6 cell surface antigens as indicators (Non-Patent Documents Nos. 2 and 3). Miyajima et al. have reported that dlk (delta-like) and Itm2A (Integral membrane protein 2A) genes are highly expressed specifically in hepatic stem cells of mouse fetal and adult livers, and that it is possible to detect or isolate hepatic stem cells using the expression of these proteins or mRNAs as indicators (Patent Document No. 1). Further, it has been also reported that isolation of rat hepatic stem cells based on the expression of specific sugar chains is possible (Patent Document No. 2).

Further, it has been reported that p75NTR which was believed to be a marker protein for stem cells in ectodermal tissues is synthesized in all kinds of tissue, and the combination of this marker with a known marker protein improves the efficiency of isolation, identification and differentiation induction of pluripotent stem cells (Patent Document No. 3).

### [Prior Art Literature]

### [Non-Patent Documents]

[Non-Patent Document No. 1] Van den Bos, C., et al, Human Cell 1997, 10,45-50
[Non-Patent Document No. 2] Suzuki A., et al, Hepatology 2000, 32:1230-39
[Non-Patent Document No. 3] Suzuki A., et al, J Cell Biology 2002, 156(1):173-84

### [Patent Documents]

[Patent Document No. 1] Japanese Unexamined Patent Publication No. 2004-187679
[Patent Document No. 2] Japanese Unexamined Patent Publication No. 2004-344031
[Patent Document No. 3] Japanese Unexamined Patent Publication No. 2006-230235

### DISCLOSURE OF THE INVENTION

### PROBLEM FOR SOLUTION BY THE INVENTION

Identification and analysis of molecular markers which recognize hepatic stem cells have progressed, and methods of isolation/culture thereof have been reported. However, such reports made to date are about rat or mouse hepatic stem cells; no reports have been made about human hepatic stem cells. The present inventors have actually confirmed that it is impossible to isolate human hepatic stem cells using conventional hepatic stem cell markers reported on mouse and rat. Thus, it has been necessary to establish an isolation method with the molecular markers specific for human hepatic stem cells.

Since drug metabolizing ability differs greatly between human liver cells and experimental animals' liver cells, it is difficult to predict the drug metabolizing ability in human liver cells from those results obtained by using experimental animals or liver cells isolated therefrom. Therefore, establishment of a technology to isolate human hepatic stem cells specifically and to differentiate/proliferate the resultant cells into human liver cells in a culture system has been demanded.

Moreover, a large number of reports made so far describe isolation methods for hepatic stem cells using a single marker. While such methods are capable of saving time and labor, they have a drawback of lacking the accuracy required for isolating only a small number of hepatic stem cells from a heterogeneous cell population.

It is an object of the present invention to provide a human hepatic stem cell.

It is another object of the present invention to provide a method of preparing the human hepatic stem cells, a method of inducing differentiation of the same, and a method of using the same.

### MEANS TO SOLVE THE PROBLEM

In the present invention, a plurality of cell surface molecules expressed in human liver cell populations have been elucidated. Then, a method of isolating human hepatic stem cells using the expression of such molecules as indicators, a method of culturing the human hepatic stem cells and a method of using the human hepatic stem cells have been established. First, as a technique of isolating human hepatic stem cells, FACS (fluorescence activated cell sorting) more accurate than MACS (magnetic cell sorting) was used. Briefly, cells were fractionated using fluorescence-labeled monoclonal antibodies to a plurality of cell surface molecules and sorted by FACS. Then, using clonal colony-forming efficiency as an indicator, those cell fractions that contain highly proliferative human hepatic stem cells exclusively and at high frequency were screened. As a result, it was confirmed in human liver cells that cells with clonal colony-forming efficiency exist at high frequency and exclusively in cell fractions having the phenotypes of CD318⁺CD90⁺CD66⁻. Clonal colonies derived from a single CD318⁺CD90⁺CD66⁻ cell expanded to 6x10⁴ colonies or more in three weeks (doubling time: 31 hrs). Since it was found that a plurality of molecular markers (albumin, cytokeratin 8/18, cytokeratin 19, desmin, SMA and vimentin) specific for liver cells, cholangiocytes and hepatic stellate cells are expressed in those cells, it has become clear that CD318⁺CD90⁺CD66⁻ cells have a pluripotency to differentiate into a plurality of different cell lineages constituting the liver.

Further, it was confirmed by semi-quantitative RT-PCR that CD318⁺CD90⁺CD66⁻ cells isolated by FACS are strongly expressing a plurality of molecular markers (AFP, Bmi-1, Dlk, CD133 and vimentin) that are expressed in hepatic stem/progenitor cells or cancer stem cells. These results indicate that hepatic stem/progenitor cells having both high proliferative capacity and pluripotency exist exclusively and at high frequency in CD318⁺CD90⁺CD66⁻ cell fractions in human fetal livers.

Further, the present inventors introduced the gene of BMI1 (one of the Polycomb group proteins which are known to be involved in replication competence that is characteristic of stem cells) into the isolated CD318⁺CD90⁺CD66⁻ cells and established a human hepatic stem cell clone. Examination of the thus established clone revealed that the clonal colony-forming efficiency thereof is extremely high.

Further, in human liver cells derived from CD318⁺CD90⁺CD66⁻ cells isolated by the above-described method, expression of a plurality of drug metabolizing enzymes (CYP3A4, 2D6, 2C9, 1A2, 2C19, 2A6, 2B6 and 2E1) and transporters (MRP2/3, MDR1 and BCRP) was confirmed by semi-quantitative PCR and an immuno-staining method. It was also confirmed that these genes are expressed similarly in human liver cells derived from a *BMI1* gene-transfected human hepatic stem cell clone.

Further, it was detected by flow cytometry analysis that CD318⁺CD90⁺CD66⁻ cells isolated by FACS were expressing CD81 tetraspanin (reported as a candidate for hepatitis C virus (HCV) receptor), scavenger receptor BI (SR-BI) and low-density lipoprotein (LDL). It was confirmed by DNA microarray analysis that the expression level of HCV receptor CD81 in FACS-sorted hepatic stem cells was significantly raised.

Further, expression of lipid metabolism-associated genes and glucogenesis-associated genes comparable to that found in human hepatic tissue was confirmed in three-dimensionally cultured transformed cells obtained by introducing *BMI1* gene into the isolated CD318⁺CD90⁺CD66⁻ cells. The expression levels of the lipid metabolism-associated genes and the glucogenesis-associated genes in the above-described three-dimensionally cultured transformed cells were confirmed to be close to their expression levels in human hepatic tissue.

Still further, the expression level of CD13 in FACS-isolated CD318⁺CD90⁺CD66⁻ cells was raised, as compared to the expression level in primary fetal liver cells.

The present invention may be summarized as follows.
(1) A human hepatic stem cell sorted based on the presence or absence of the expression of at least one marker selected from the group consisting of CD318, CD90, CD66 and CD 13.
(2) The human hepatic stem cell according to (1), which has the phenotype of CD318⁺.
(3) The human hepatic stem cell according to (1) or (2), which has the phenotype of CD90⁺.
(4) The human hepatic stem cell according to any one of (1) to (3), which has the phenotype of CD66⁻.
(5) The human hepatic stem cell according to (1), which has the phenotypes of CD318⁺CD90⁺CD66⁻.
(6) The human hepatic stem cell according to (5), which is a cell clone LSC-E2 deposited at the International Patent Organism Depository, National Institute of Advanced Industrial Science and Technology, Japan under accession number FERM BP-11108.
(7) The human hepatic stem cell according to any one of (1) to (6), which has the phenotype of CD13⁺.
(8) The human hepatic stem cell according to (1), which is a cell, or a progeny thereof, that has been obtained by proliferating into a cell population, a human hepatic stem cell clone sorted based on the presence or absence of the expression of at least one marker selected from the group consisting of CD318, CD90, CD66 and CD13 and then sorting cells from the cell population based on the presence or absence of the expression of at least one marker selected from the group consisting of CD318, CD90, CD66 and CD13.
(9) A transformed cell, wherein *BMI1* gene has been introduced into the human hepatic stem cell according to (1).
(10) A method of preparing the human hepatic stem cell according to (1), which comprises sorting cells from a human liver cell population based on the presence or absence of the expression of at least one marker selected from the group consisting of CD318, CD90, CD66 and CD13.
(11) The method according to (10), wherein the human liver cell population is a primary liver cell culture line isolated from a human liver.
(12) The method according to (10), wherein after sorting cells from a human liver cell population based on the presence or absence of the expression of at least one marker selected from the group consisting of CD318, CD90, CD66 and CD13, the resultant cells are proliferated into a cell population and then cells are sorted again from this cell population based on the presence or absence of the expression of at least one marker selected from the group consisting of CD318, CD90, CD66 and CD 13.
(13) The method according to (12), wherein cell sorting and proliferation are repeated twice or more.
(14) A method of preparing a liver cell which expresses drug-metabolizing enzymes and/or transporters at the protein level, the method of comprising inducing the differentiation of the human hepatic stem cell according to (1) and/or the transformed cell according to (9).
(15) The method according to (14), wherein the differentiation of the human hepatic stem cell according to (1) and/or the transformed cell according to (9) is induced by treatment with an extracellular matrix and/or a growth factor.
(16) The method according to (15), wherein the extracellular matrix and/or growth factor is at least member of the group consisting of laminin, type I collagen, type IV collagen, fibronectin, metrigel, dexamethasone, DMSO, oncostatin M, insulin, HGF, EGF, TGFα, HB-EGF, VEGF and PDGF.
(17) The method according to (14), wherein the differentiation of the human hepatic stem cell according to (1) and/or the transformed cell according to (9) is induced by three-dimensional culture.
(18) A liver cell expressing drug-metabolizing enzymes and/or transporters at the protein level, which has been prepared by the method according to (14)
(19) The liver cell according to (18), wherein the drug-metabolizing enzyme is at least one member of the group consisting of CYP3A4, CYP2C9, CYP2C19, CYP2D6, CYP2B6, CYP2E1 and CYP2A6.
(20) The liver cell according to (18), wherein the transporter is at least one member of the group consisting of ABC2, ABCA6, ABCA8, MDR1, MDR3, BSEP, MRP1, MRP2, MRP5, MRP6, ABCG8, NTCP, PEPT1, OATP-C, OATP8, OATP-B, OATP-F, OCT1, OAT2, SLC22A18, CNT1, ENT1, MATE 1, MRP3 and BCRP.
(21) A method of evaluating the metabolism and/or transportation of a drug candidate compound using the liver cell according to (18).
(22) A bioreactor comprising the human hepatic stem cell according to (1), the transformed cell according to (9) and/or a liver cell which has been induced to differentiate therefrom.
(23) The bioreactor according to (22), which is used for as an artificial liver.
(24) A human model cell for experiments on HCV infection and/or inhibition of HCV replication, comprising the human hepatic stem cell according to (1), the transformed cell according to (9) and/or a liver cell which has been induced to differentiate therefrom.
(25) A human model cell for glucose metabolism experiments, comprising the human hepatic stem cell according to (1), the transformed cell according to (9) and/or a liver cell which has been induced to differentiate therefrom.
(26) A human model cell for lipid metabolism experiments, comprising the human hepatic stem cell according to (1), the transformed cell according to (9) and/or a liver cell which has been induced to differentiate therefrom.
(27) A method of using in regenerative medicine with the human hepatic stem cell according to (1), the transformed cell according to (9) and/or a liver cell which has been induced to differentiate therefrom.
(28) The method according to (27), wherein the regenerative medicine is cell therapy.

### EFFECT OF THE INVENTION

According to the present invention, it has become possible to isolate human hepatic stem cells more accurately because of the discovery of human hepatic stem cell markers as opposed to the conventional mouse or rat cell markers. The present inventors have actually confirmed that it is impossible to specifically detect human liver cells with conventional mouse hepatic stem cell markers. Thus, the significance of the present invention is great.

Further, since antigen-antibody reactions are used for cell isolation, the background level is smaller than in the methods using mRNAs or sugar chains as indicators. Thus, in the present invention, it is possible to isolate hepatic stem cells specifically as opposed to conventional methods.

For industrial application as in drug development, human liver cells retaining a maturing function are needed. It was confirmed that human liver cells induced to differentiate from human hepatic stem cells using the above-described technology were expressing various drug-metabolizing enzymes (CYPs) and transporters. Such human liver cells are highly valuable in performing screenings for drug development in such aspects as drug metabolism.

The present specification encompasses the contents disclosed in the specification and/or the drawings of Japanese Patent Application No. 2008-127755 based on which the present patent application claims priority.

### BRIEF DESCRIPTION OF THE DRAWINGS

Fig. 1A Clonal colony-forming efficiency of human primary fetal liver cells in low-density culture
Fig. 1B Bi-potential differentiation capabilities of human primary fetal liver cells *in vitro*
Fig. 1C Differentiation capabilities of human primary fetal liver cells *in vitro* (glycogen storage capacity)
Fig. 1D Differentiation capabilities of human primary fetal liver cells *in vitro* (gene expression)
Fig. 1E Surface antigen profiling of human primary fetal liver cells using flow cytometry
Fig. 2A Isolation of human hepatic stem cells using flow cytometry
Fig. 2B Phenotypes of clonal colonies in individual cell fractions
Fig. 2C Hepatic stem cell-derived clonal colonies
Fig. 2D Ability to form single cell-derived clonal colonies *in vitro* (quantitative analysis)
Fig. 2E Tracking observation of hepatic stem cells *in vitro*
Fig. 2F Expansion curve of hepatic stem cells *in vitro*
Fig. 3A Gene expression analysis in hepatic stem cell-derived clonal colonies
Fig. 3B Expression analysis of hepatocyte and cholangiocyte markers in hepatic stem cell-derived clonal colonies
Fig. 3C Glycogen storage capacity of hepatic stem cells
Fig. 3D Long-term culture of and CYP450 expression in hepatic stem cells
Fig. 4A Phenotype in *BMI1* overexpressing hepatic stem cells
Fig. 4B Clonal colony-forming efficiency enhanced by *BMI1* gene transfection
Fig. 4C Differentiation capabilities of *BMI1* overexpressing human hepatic stem cells (glycogen storage capacity)
Fig. 4D Expression of cytochrome P450 in *BMI1* overexpressing human hepatic stem cells (immunocytochemistry)
Fig. 4E Gene expression analysis in *BMI1* overexpressing human hepatic stem cells
Fig. 5 Establishment of human hepatic stem cell
Fig. 6 Strategy of *BMI1* transfection
Fig. 7A Culture of human liver cells in three-dimensional culture system
Fig. 7B Three-dimensional culture of *BMI1*-transfected cells
Fig. 7C Gene expression analysis of liver cells using two-dimensional and three-dimensional culture systems
Fig. 7D Enhanced gene expression in three-dimensional culture system
Fig. 7E Gene expression analysis of *BMI1*-transfected hepatic stem cells in three-dimensional culture system
Fig. 8 Analysis of HCV receptors of human hepatic stem cells using flow cytometry
Fig. 9 Expression analysis of lipid metabolism-associated genes and glucogenesis-associated genes in human liver cells which were induced to differentiate from hepatic stem cells

### BEST MODE FOR CARRYING OUT THE INVENTION

Hereinbelow, the embodiment of the present invention will be described in detail.

The present invention provides a human hepatic stem cell sorted based on the presence or absence of the expression of at least one marker selected from the group consisting of CD318, CD90, CD66 and CD 13. CD318 is an antigen molecule expressed in hematopoietic stem cells, mesenchymal stem cells, neural stem cells, etc. CD90 is an antigen molecule expressed in neural tissues, connective tissues, stromal cells, etc., and expression of this antigen molecule is also recognized in hematopoietic stem cells, mesenchymal cells and hepatic progenitor cells. CD66 has been reported as a granulocyte-specific antigen associated with carcinoembryonic antigen found in colon cancer cells and is an immunoglobulin super family molecule. CD13 is one of the mesenchymal stem cell markers. The human hepatic stem cell of the present invention may have at least one phenotype selected from CD318⁺, CD90⁺, CD66- and CD13⁺. The human hepatic stem cell of the present invention preferably has the phenotypes of CD318⁺CD90⁺CD66⁻, and more preferably the phenotypes of CD318⁺CD90⁺CD66⁻CD13⁺.

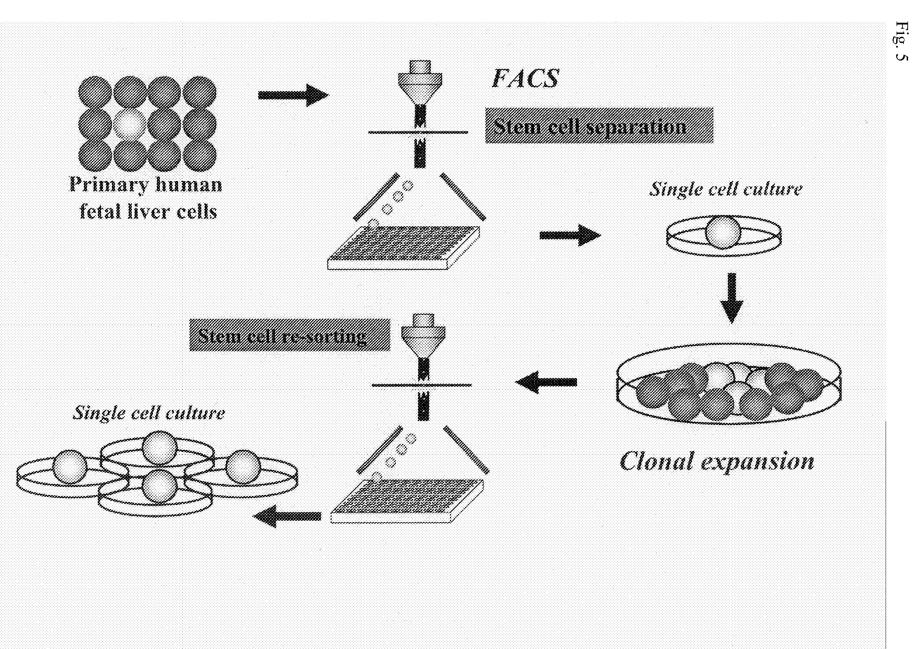

The human hepatic stem cell of the present invention is obtained by sorting cells of interest from a human liver cell population based on the presence or absence of the expression of at least one marker selected from the group consisting of CD318, CD90, CD66 and CD13. The present invention also provides a method of preparing a human tissue stem cell, which comprises sorting cells of interest based on the presence or absence of the expression of at least one marker selected from the group consisting of CD318, CD90, CD66 and CD 13. The human liver cell population may be a population of any human liver cells. For example, a primary liver cell culture line isolated from a human liver (e.g., human fetal liver cells, Cat No. CS-ABI-3716, Cell Systems) may be given. After cells of interest are sorted from a human liver cell population based on the presence or absence of the expression of at least one marker selected from the group consisting of CD318, CD90, CD66 and CD13, the resultant cells are expanded into a cell population. From this cell population, cells of interest may be sorted again based on the presence or absence of the expression of at least one marker selected from the group consisting of CD318, CD90, CD66 and CD 13 (Fig. 5). Cell sorting may be performed by any method such as MACS (magnetic cell sorting) or FACS (fluorescence activated cell sorting). Since FACS is capable of sorting and isolating cells with high purity, use of FACS is preferable. Culture of the thus sorted cells may be performed for about 2 to 3 weeks or longer in a medium such as a mixed medium of DMEM and F-12 Ham, William's medium E or RPMI1640, each supplemented with FBS (10%), insulin (1 µg/ml), dexamethasone (10⁻⁷M), HGF (50 ng/ml), EGF (20 ng/ml) or the like. Exchange of the medium is preferably carried out every five days. For culturing cells, it is preferable to use a three-dimensional culture system using a culture substratum for three-dimensional culture, collagen gel, or the like. Cell sorting and proliferation may be repeated twice or more. After clone sorting with FACS, a single cell is cultured separately, followed by selection of those cells with clonal colony-forming efficiency. By these procedures, it is possible to establish a human tissue stem cell clone. One of the thus established cell clones was designated LSC-E2 and deposited at the International Patent Organism Depository, National Institute of Advanced Industrial Science and Technology (located at Central 6, 1-1-1 Higashi, Tsukuba City, Ibaraki Pref., Japan; zip code: 305-8566) under accession number FERM BP-11108 (date of accession: April 3, 2008). It should be noted that this deposit was transferred from FERM P-21563 which was deposited on April 3, 2008 (domestic date of accession). Clone LSC-E2 has the phenotypes of CD318⁺CD90⁺CD66⁻. Further, the thus established clone (e.g., LSC-E2) may be proliferated into a cell population, and cells of interest may be sorted from this cell population based on the presence or absence of the expression of at least one marker selected from the group consisting of CD318, CD90, CD66 and CD13.

The human hepatic stem cell of the present invention has a high proliferative capacity and a potency to differentiate into liver cells and bile duct epithelial cells. The human hepatic stem cell of the present invention and cells which have been induced to differentiate therefrom are expected to be used as artificial livers or in regenerative medicine such as cell transplantation. With respect to the artificial liver, hybrid-type artificial livers consisting of a combination of liver cells and artificial devices are mainly used at present. As one example, an artificial liver bioreactor may be given which is prepared by attaching pig liver cells to fine beads approx. 2 mm in diameter and placing the resultant beads outside of the hollow fibers of a bioreactor composed of a bundle of hollow, semi-transparent membrane fibers (hollow fibers). This bioreactor has been clinically tested and achieved success to some extent. The human hepatic stem cell of the present invention and cells which have been induced to differentiate therefrom are applicable to bioreactors including artificial livers. Such bioreactors are not only used as artificial livers but also applicable to production of physiologically active substances such as human albumin and blood coagulation factors, assessment of the metabolism and transportation of drug candidate compounds, experiments on HCV infection and inhibition of HCV replication, experiments on glucose metabolism, experiments on lipid metabolism, and so forth. It is possible to create a bioreactor with a liver function by packing a matrix (such as artificial material) with the human hepatic stem cell of the present invention and cells which have been induced to differentiate therefrom.

It is possible to use the human hepatic stem cell of the present invention in regenerative medicine such as cell transplantation. Briefly, it is possible to perform orthotopic or heterotopic cell transplantation by injecting the human hepatic stem cell of the present invention into the spleen, the portal vein, the mesentery or the peritoneal cavity of immunosuppressant-administered patients with severe liver failure. Alternatively, it is possible to perform orthotopic or heterotopic cell transplantation by packing a matrix (such as artificial material) with the human hepatic stem cell of the present invention and cells which have been induced to differentiate therefrom and injecting the matrix into the spleen, the portal vein, the mesentery or the peritoneal cavity of patients with severe liver failure.

Further, since it was confirmed that those cells which have been induced to differentiate from the human hepatic stem cell of the present invention are expressing various drug-metabolizing enzymes (CYPs) and transporters, those cells may be used in screening for drug development (as in drug metabolism tests).

Further, since it was confirmed that the human hepatic stem cell of the present invention is expressing CD81 tetraspanin (reported as a candidate for HCV receptor), scavenger receptor BI (SR-BI) and low-density lipoprotein receptor (LDLR), the cell may be used as a human model cell for experiments of HCV infection and inhibition of HCV replication. Moreover, it was confirmed that the human hepatic stem cell of the present invention and cells which have been induced to differentiate therefrom are expressing lipid metabolism-associated genes and glucogenesis-associated genes. Therefore, those cells may be used as human model cells for lipid metabolism experiments and glucose metabolism experiments.

No culture system has yet been established for HCV-infected cells (excluding some HCV clones). Therefore, the mode of HCV infection has not bee elucidated yet. Except for chimpanzee, no HCV-sensitive experimental animals have been confirmed. Therefore, development of vaccines and therapeutics is extremely difficult. Briefly, the most important point in HCV researches is to develop a highly reproducible and efficient culture system. When establishment of a culture cell line of the hepatic stem cell of the present invention which is capable of highly expressing HCV binding receptors such as hCD81 and SR-B1 becomes possible by three-dimensional culture system, then it becomes possible to perform HCV infection experiment, whereby the fusion between viral membrane and cellular membrane (which is most the important in viral infection) and the mechanism of invasion of the virus genome into cells can be investigated. Such a culture cell line is also applicable to drug development as in screening for blockers of new pathways of HCV infection routes. Further, it is also expected to use such a culture cell line in the development of therapeutic antibodies such as binding preventive antibodies against HCV receptors, virus-neutralizing antibodies and human type antibodies to HCV envelope proteins and in the activity assessment of human type antibodies.

Since it was confirmed that the human hepatic stem cell of the present invention and cells which have been induced to differentiate therefrom are expressing lipid metabolism-associated genes and glucogenesis-associated genes, these cells may be used as human model cells for lipid metabolism experiments and glucose metabolism experiments. With these cells, screening for various drugs associated with lipid metabolism and glucose metabolism may be performed. Briefly, it is possible to predict the effect of a drug which promotes or inhibits lipid metabolism or glucogenesis by adding the drug of interest into the culture broth.

The present invention also provides a transformed cell obtained by introducing *BMI1* gene into a human hepatic stem cell sorted based on the presence or absence of the expression of at least one marker selected from the group consisting of CD318, CD90, CD66 and CD13. BMI1 is one of the Polycomb group proteins which are known to be involved in replication competence of cells and the genetic information thereof is registered at NCBI GenBank (GenBank accession number: NM_005180.5).

The transformed cell of the present invention may be prepared as described later in the Example. However, one of ordinary skill in the art could appropriately add alterations and modifications to the preparation method disclosed.

It has been found that introduction of *BMI1* gene into the stem cell of the present invention enhances its clonogenicity. Further, it was confirmed that the genes of various drug-metabolizing enzymes (CYPs) and transporters expressed in those cells which have been induced to differentiate from the stem cell of the present invention are also expressed similarly in the *BMI1*-transfected human hepatic stem cell. In particular, it was confirmed that the expression level of *CYP3A4* gene was raised compared to the level in the hepatic stem cell without *BMI1*-transfection. Therefore, it is believed that the *BMI1*-transfected human hepatic stem cell may also be used in drug development studies such as drug metabolism experiments. Further, it was confirmed that the lipid metabolism-associated genes and glucogenesis-associated genes expressed in those cells which have been induced to differentiate from the stem cell of the present invention are also expressed similarly in the *BMI1*-transfected human hepatic stem cell. The expression levels of such genes were confirmed to be close to their expression levels in human tissues. Therefore, it is believed that the *BMI1*-transfected human hepatic stem cell may also be used as human model cell for lipid metabolism experiments and glucose metabolism experiments. It has become possible to culture the *BMI1-*transfected human hepatic stem cell without using extracellular matrix-coated dishes which are essential for conventional liver cell culture systems. The *BMI1-*transfected human hepatic stem cell is also applicable to artificial livers, regenerative medicine such as cell transplantation, bioreactors, etc. and may also be used as a human model cell in experiments on HCV infection and inhibition of HCV replication.

Further, the present invention provides a method of preparing a liver cell which expresses drug-metabolizing enzymes and/or transporters at the protein level, the method comprises inducing the differentiation of a human hepatic stem cell sorted based on the presence or absence of the expression of at least one marker selected from the group consisting of CD318, CD90, CD66 and CD13 and/or a transformed cell wherein *BMI1* gene has been introduced into the human hepatic stem cell. Specific examples of drug-metabolizing enzymes include, but are not limited to, CYP3A4, CYP2C9, CYP2C19, CYP2D6, CYP2B6, CYP2E1 and CYP2A6. Specific examples of transporters include, but are not limited to, MRP2, MRP3, MDR1 and BCRP. For example, one or two or more transporters selected from the following may be expressed: ABC2, ABCA6, ABCA8, MDR1, MDR3, BSEP, MRP1, MRP2, MRP5, MRP6, ABCG8, NTCP, PEPT1, OATP-C, OATP8, OATP-B, OATP-F, OCT 1, OAT2, SLC22A18, CNT 1, ENT1 and MATE1.

The differentiation into a liver cell which expresses drug-metabolizing enzymes and/or transporters at the protein level may be induced by treating with an extracellular matrix and/or a growth factor, when a human hepatic stem cell sorted based on the presence or absence of the expression of at least one marker selected from the group consisting of CD318, CD90, CD66 and CD 13 and/or a transformed cell wherein *BMI1* gene has been introduced into the human hepatic stem cell. Specific examples of the extracellular matrix and/or the growth factor include, but are not limited to, laminin, type I collagen, type IV collagen, fibronectin, metrigel, dexamethasone, DMSO, oncostatin M, insulin, HGF, EGF, TGFα, HB-EGF, VEGF and PDGF. The treatment with an extracellular matrix and/or a growth factor may be performed by culturing a human tissue stem cell with the phenotypes of CD318⁺CD90⁺CD66⁻ in a medium containing the extracellular matrix molecule and/or the growth factor. Specific examples of the medium include, but are not limited to, a mixed medium of DMEM and F-12 Ham, William's medium E and RPMI1640 medium. Culture may be continued until cells reached 90% confluence in culture dishes. When cells reached 90% confluence, passage may be carried out as described below. Briefly, the culture medium is removed; the cells are treated with 0.05% trypsin-EDTA at room temperature for 5 min and gently detached from dishes by tapping; the suspended cells are washed with a culture medium containing 10% FBS and then replated in a culture medium.

Alternatively, the differentiation into a liver cell which expresses drug-metabolizing enzymes and/or transporters at the protein level may be induced by three-dimensional culture of a human hepatic stem cell sorted based on the presence or absence of the expression of at least one marker selected from the group consisting of CD318, CD90, CD66 and CD13 and/or a transformed cell wherein *BMI1* gene has been introduced into the human hepatic stem cell. For three-dimensional culture of cells, it is preferable to use a three-dimensional culture system using a culture substratum for three-dimensional culture, collagen gel, or the like.

The present invention also provides a liver cell expressing drug-metabolizing enzymes and/or transporters at the protein level, which has been obtained by inducing differentiation of a human hepatic stem cell sorted based on the presence or absence of the expression of at least one marker selected from the group consisting of CD318, CD90, CD66 and CD 13 and/or a transformed cell wherein *BMI1* gene has been introduced into the human hepatic stem cell. Specific examples of drug-metabolizing enzymes include, but are not limited to, CYP3A4, CYP2C9, CYP2C19, CYP2D6, CYP2B6, CYP2E1 and CYP2A6. Specific examples of transporters include, but are not limited to, MRP2, MRP3, MDR1 and BCRP. For example, one or two or more transporters selected from the following may be expressed: ABC2, ABCA6, ABCA8, MDR1, MDR3, BSEP, MRP1, MRP2, MRP5, MRP6, ABCG8, NTCP, PEPT1, OATP-C, OATP8, OATP-B, OATP-F, OCT1, OAT2, SLC22A18, CNT1, ENT1 and MATE1. It is possible to evaluate the metabolism and/or transportation of drug candidate compounds using the liver cell of the present invention which expresses drug-metabolizing enzymes and/or transporters at the protein level.

Therefore, the present invention also provides a method of evaluating the metabolism and/or transportation of drug candidate compounds using the above-described liver cell expressing drug-metabolizing enzymes and/or transporters at the protein level. As one example, a drug candidate compound may be added to a cultured liver cell that expresses drug-metabolizing enzymes and/or transporters at the protein level and then examined for its metabolism and/or transportation. The metabolism and/or transportation of drug candidate compounds may be determined by such methods as mass spectrometry and HPLC analysis. The drug candidate compound may be any substance. For example, proteins, peptides, vitamins, hormones, polysaccharides, oligosaccharides, monosaccharides, low molecular weight compounds, nucleic acids (DNA, RNA, oligonucleotides, mononucleotides, etc.), lipids, natural compounds other than those listed above, synthetic compounds, plant extracts, fractionated products from plant extracts, and mixtures thereof may be enumerated.

### EXAMPLES

Hereinbelow, the present invention will be described more specifically with reference to the following Example. However, the present invention is not limited to this Example.

### [Example 1]

### Materials and Methods

### Source of Human Liver Cells

The human fetal liver cells used in the present study were isolated by ACBRI (Applied Cell Biology Research Institute, a registered Washington non-profit research institution) in the US under the consent of the supplier and provided for free by Cell Systems in the US (Cat No. CS-ABI-3716). In Japan, the human fetal liver cells are supplied by Dainippon Pharmaceutical as Human Primary Fetal Hepatocytes (Cat No. CS-ABI-3716). This product was used in the present study. The results of infection tests (with HIV, HBV and HCV) and tests on microorganisms (fungi, bacteria and Mycoplasma) were negative. The present study has been deliberated and approved at the ethics committee of the university to which the present inventors belong.

### Cell Culture and Passage

The modifications as described below were made to an already established and reported low-density culture system for clonogenic fetal liver cells (Zheng YW, Taniguchi H, et al. Transplant Proc 2000;32:2372-2373; Suzuki A, Zheng YW, et al. Hepatology 2000;32:1230-1239).

The primary fetal liver cells, cells fractionated by flow cytometry or *BMI1*-transfected transformed cells were plated in DMEM nutrient mixture F-12 Ham (DMEM/F12 1:1 mixture, SIGMA) supplemented with 10% fetal bovine serum (FBS), human γ-insulin (1.0 µg/ml, Wako, Japan), nicotinamide (10 mM, SIGMA), dexamethasone (1x10-⁷M, SIGMA) and L-glutamine (2 mM, GIBCO BRL) in type IV collagen-coated dishes (Becton Dickinson Labware) and cultured for about two to three weeks or longer, with complete medium change performed every five days. Growth factors such as human recombinant HGF (50 ng/ml, SIGMA) and epidermal growth factor (EGF) (10 ng/ml, SIGMA) were added 24 hours after the plating.

When cells reached 90% confluence in culture dishes, passage was performed as described below. Briefly, the culture medium was removed; the cells were treated with 0.05% trypsin-EDTA at room temperature for 5 min and gently detached from dishes by tapping; the suspended cells were washed with a culture medium containing 10% FBS and then replated in a culture medium. The results of trypan blue staining revealed that the viability of dissociated cells was never lower than 90%. Depending on the experiment design, the plating density of cells was determined from single cell culture (a technique of culturing a single flow-cytometrically sorted cell in one well of a 96-well plate), low density culture at 100-500 cells/cm² or high density culture at 1x10³ cells/cm².

### Clonal Colony Assay

Flow cytometric sorting and single cell-based assays were performed in this study. For assessing the clonogenic and proliferative capacities of flow cytometrically fractionated cells, hepatocyte clonal colony-forming units in culture (H-CFU-C) and the relative frequency of H-CFU-C formation in plated cells were determined after two- to three-week culture. H-CFU-C is defined as a unit capable of forming a colony containing 500 or more cells derived from a single cell capable of proliferation in vitro.

### Cell Profiling and Sorting by Flow Cytometry

Suspended cells were incubated on ice with fluorochrome-conjugated monoclonal antibodies (mAbs) at optimized concentrations for 30 min in the dark. 2% FBS-supplemented PBS was used as a washing solution and an antibody diluent. When biotinylated primary antibody was used, secondary reaction was performed with streptavidin-labeled fluorescent antibody. All fluorochrome-conjugated mAbs were purchased from Becton Dickinson excepting those listed below: fluorescein isothiocyanate (FITC)-conjugated anti-human CD66 (hCD66FITC), allophycocyanin (APC)-conjugated hCD90, phycoerythrin (PE)-conjugated hCD318, hCD24FITC, hCD29FITC, hCD44PE, hCD49fPE, biotinylated hCD54 (eBioscience); hCD117APC, hCD138PE (Miltenyi Biotec), hCD140aPE and Streptavidin-labeled APC. Cell profiling analyses and sorting were performed on a high speed cell sorter, MoFlo (DakoCytomation).

### Cytochemical and Immunocytochemical Assays

PAS stain (periodic acid-Schiff stain): Periodic acid-Schiff stain was performed to determine the glycogen synthesis and storage in cultured cells.

In dual or triple immunocytochemical staining, cells were fixed with cold ethanol for 30 min and then blocked with 10% normal goat serum (NGS) for 60 min. Subsequently, a primary antibody was diluted with 1% NGS-supplemented PBS and reacted with the cells in a moist chamber at 4°C overnight. A secondary antibody was diluted with 10% glycerol-containing PBS and reacted with the cells in a moist chamber at room temperature for 60 min. The cell nuclei were counter-stained with DAPI and mounted with an FA mounting fluid. (Images were captured with Zeiss Axiolmager microscope.)

Primary antibodies used in innunocytochemical assays were mouse anti-human albumin mAb (SIGMA), mouse anti-human CK19 mAb (Progen), and rabbit anti-human CYP 3A4 (Chemicon), CYP 2D6 (Chmicon), CYP 2C9 (Daiichi Pure Chemicals; Tokyo). Secondary antibodies used in the assays were Alexa488-conjugated goat anti mouse IgG, Alexa555-conjugated goat anti mouse IgG, Alexa647-conjugated goat anti mouse IgG and Alexa488-conjugated goat anti-rabbit IgG (Invitrogen, Molecular Probes).

### Semi-Quantitative RT-PCR and Real Time PCR

Total RNA from cells or cell colonies was extracted using Isogen reagent (Nippon Gene, Toyama, Japan). Before reverse transcription (RT), 150 ng of random primers and 1 µl of 10mM dNTP mix were added to the total RNA solution. The reaction mixture was heated at 65°C for 5 min and incubated on ice for 1 minute. Subsequently, 1x first-strand buffer, 0.5 mM dNTP mix, 5 mM DTT and 200 units of Super Script III (Invitrogen) were added to the reaction mixture, which was then incubated at 25°C for 5 min, at 50°C for 45 min and at 70°C for 15 min to thereby synthesize cDNA from the total RNA. Semi-quantitative PCR was performed in 20 µl of reaction mixture (1x PCR buffer, Taq DNA polymerase) (Takara Shuzo Co., Tokyo, Japan). PCR was performed using hepatocyte-specific primers for the following: albumin [5'-tgttgattgcctttgctcag-3' (SEQ ID NO: 1) and 5'-tggagactggcacacttgag-3'(SEQ ID NO: 2)]; AFP [5'-agcttggtggtggatgaaac-3' (SEQ ID NO: 3) and 5'-ccctcttcagcaaagcagac-3' (SEQ ID NO: 4)]; G6P [5'-gtcaacacattacctccagg-3' (SEQ ID NO: 5) and 5'-gagtagatgtgaccatcacg-3' (SEQ ID NO: 6)]; CYP3A4 [5'-aacagcctgtgctggctatc-3' (SEQ ID NO: 7) and 5'-gatcacatccatgctgtagg-3' (SEQ ID NO: 8)]; CYP2C9 [5'-gcaggaaaacggatttgtgt-3' (SEQ ID NO: 9) and 5'-caggccatctgctcttcttc-3' (SEQ ID NO: 10)]; CYP2C19 [5'-cttcaccctgtgatcccact-3' (SEQ ID NO: 11) and 5'-aaagtcccgagggttgttg-3' (SEQ ID NO: 12)]; CYP2D6 [5'- aggaggagtcgggctttctg-3' (SEQ ID NO: 13) and 5'-cttggccttctccatctctg-3' (SEQ ID NO: 14)]; CYP1A2 [5'-cagaatgccctcaacacctt-3' (SEQ ID NO: 15) and 5'-gctcctggactgttttctgc-3'(SEQ ID NO: 16)]; CYP2B6 [5'-aacctgcaggaaatcaatgc-3' (SEQ ID NO: 17) and 5'-attttggctcggtcatgaag-3' (SEQ ID NO: 18)]; CYP2E1 [5'-acccgagacaccattttcag-3' (SEQ ID NO: 19) and 5'-agggtgtcctccacacactc-3' (SEQ ID NO: 20)], for GAPDH [5'-ctctgctcctcctgttcgac-3' (SEQ ID NO: 21) and 5'-ttgattttggagggatctcg-3' (SEQ ID NO: 22)]; EGFP [5'-ctacggcgtgcagtgcttca -3' (SEQ ID NO: 23) and 5'-cgctgccgtcctcgatgttg -3' (SEQ ID NO: 24)]; BMI1 [5'-aatccccacctgatgtgtgt-3' (SEQ ID NO: 25) and 5'-catttttgaaaagccctgga-3' (SEQ ID NO: 26)]; TAT [5'-ttctggcagtggctgcac-3' (SEQ ID NO: 27) and 5'-cccatctcggatctcattgc-3' (SEQ ID NO: 28)], for GULU [5'-aaggcatcaagcaggtgtac-3' (SEQ ID NO: 29) and 5'-gctggttgctcaccatgtcc-3' (SEQ ID NO: 30)]; GGT [5'-acaaccatgtgtacaccagg-3' (SEQ ID NO: 31) and 5'-ctggtgtgccagctcatacg-3' (SEQ ID NO: 32)]; Desmin [5'-aagctgcaggaggagattca-3' (SEQ ID NO: 33) and 5'-ggcagtgaggtctggcttag-3' (SEQ ID NO: 34)]; CYP2A6 [5'-agaggaagagcaaggggaag-3' (SEQ ID NO: 35) and 5'-ctgaactcctcagcctggtc-3' (SEQ ID NO: 36)]; MRP2 [5'-tgcttcctggggataatcag -3' (SEQ ID NO: 37) and 5'-cacggataactggcaaacct-3' (SEQ ID NO: 38)]; MDR1 [5'-gctcctgactatgccaaagc-3' (SEQ ID NO: 39) and 5'-tcttcacctccaggctcagt-3' (SEQ ID NO: 40)]; MRP3 [5'-tgtgctaggtgatggacagg-3' (SEQ ID NO: 41) and 5'-tgtcacctgcaccttctctg-3' (SEQ ID NO: 42)]; BCRP [5'-ttttctgttcccttgccatc-3' (SEQ ID NO: 43) and 5'-cctggccctctactctaccc-3' (SEQ ID NO: 44)]; PROM1 [5'-tccaacagggctatcaatcc-3' (SEQ ID NO: 45) and 5'-ccaaagacaaaggtaagaaccac-3' (SEQ ID NO: 46)]; CD24 [5'-cccatcttctaaacccaatcc-3' (SEQ ID NO: 47) and 5'-cattccacaatcccatcctt-3' (SEQ ID NO: 48)]; CD44 [5'-tttacagcctcagcagagca-3' (SEQ ID NO: 49) and 5'-cgatgctcagagctttctcc-3' (SEQ ID NO: 50)]; ALCAM [5'-cgtctgctcttctgcctctt-3' (SEQ ID NO: 51) and 5'-tggttgcttgaacaccttga-3' (SEQ ID NO: 52)]; and EPCAM [5'-tgatcctgactgcgatgaga-3' (SEQ ID NO: 53) and 5'-gcacaacaattccagcaaca-3' (SEQ ID NO: 54)]. PCR conditions were as follows: initial denaturation at 94°C for 30 sec, annealing at 60°C for 30 sec and extension at 72°C for 30 sec. For GAPDH, annealing temperature was 57°C; for EGFP and BMI1, annealing temperature was 64°C; and for TAT, annealing temperature was 65°C. PCR products were electrophoresed in 1.5% agarose gel.

Taqman probes and primers for albumin (Hs00609411_ml), CYP3A4 (Hs01546612_ml), CYP2C9 (Hs00426397_ml), CYP2D6 (Hs02576167_ml) and 18S rRNA (4319413E) were obtained from TaqMan Gene Expression Assays (Applied Biosystems).

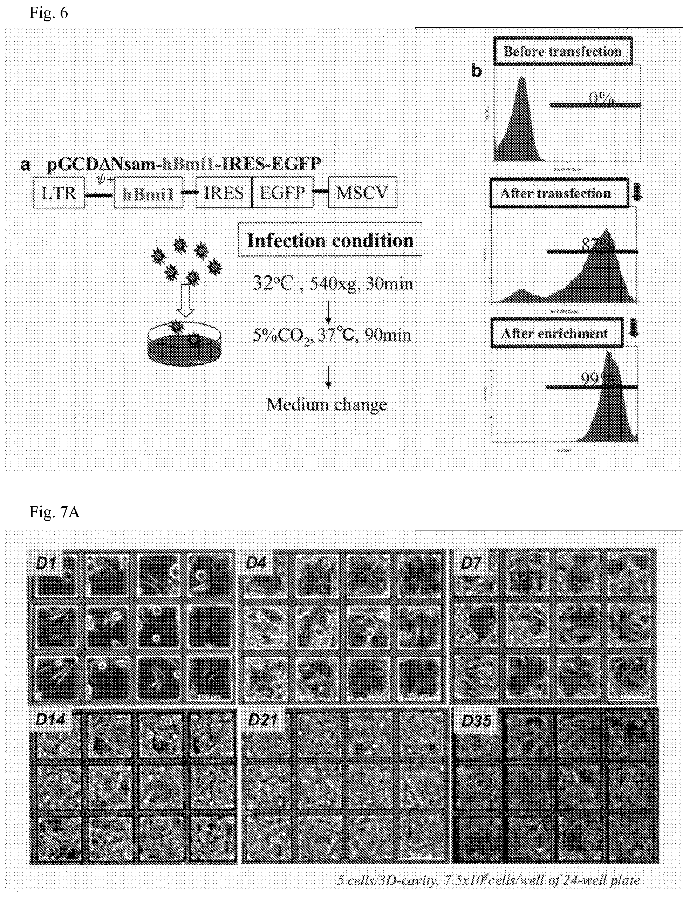

### Preparation of Retrovirus Vector and Establishment of BMI1-Transfected Hepatic Stem Cell Clone (Fig.6)

For preparing the retrovirus vector used in the present study, a retrovirus vector pGCDNsam IRES-EGFP (Nabekura T, Otsu M, et al. Mol Ther. 2006; 13(2):301-9) with which stable and long-term expression of the introduced gene can be expected and packaging cell strains (293GP and 293GPG cells) (provided by Prof. Makoto Ohtsu, Center for Stem Cell Biology and Regenerative Medicine, the Institute of Medical Science, the University of Tokyo) in which virus vector production can be induced by using a tetracycline-inducible system (TET-ON/OFF) were used (Bums JC, Friendmann T, et al. Proc. Natl. Acad. Sci. USA 1993, 90: 8033-7; Ory DS, Neugeboren BA, et al. Proc Natl Acad Sci U S A. 1996, 93:11400-6).

The cDNA of *BMI1* cloned from a human fetal liver was integrated into pGCDNsam IRES-EGFP vector at the multi-cloning site (NotI-BamHI) upstream of IRES domain. The resultant vector was introduced into the packaging cell (293GP cells) with calcium phosphate reagent (Promega). Subsequently, 293GPG cells were infected with the produced virus vector to thereby establish a stable expression strain under the condition of TET-ON.

These cells were cultured under the condition of TET-OFF. The resultant culture supernatant containing the produced virus vector was concentrated by centrifugation (6000xg, 4°C, 16 hr) and suspended in StemPro-34 Serum Free Medium (Invitrogen). The titer of the resultant virus vector was confirmed with Jurkat cells (RIKEN BRC) (7.9 x 10⁶ infectious units/ml).

Culture of these packaging cell strains was performed in DMEM medium containing 10% FBS (Gibco), 2 mmol/L L-glutamine (Gibco), 2 µg/ml Puromycin (Sigma), 0.3 mg/ml G418 (Sigma) and 1x penicillin/streptomycin (Gibco) at 37°C under 10% CO₂. For culture dishes, those coated with poly-L-lysine were used.

Subsequently, for extablishing a *BMI1*-transfected hepatic stem cell clone, the virus vector was introduced into human hepatic stem cells one week after their plating in the presence of protamin sulfate (10 µg/ml, Wako), followed by centrifugation (540xg, 32°C, 30 min) and incubation (37°C, 5% CO₂, 1.5 hr). Seventy-two hours later, *BMI1*-transfected cells were enriched by flow cytometry (EGFP positive ratio: 99%).

### Three-Dimensional Culture

Type IV collagen (Nitta Gelatin, Osaka) was coated on the surface of a micropattern cell culture film (PMMA/EH; provided by Kuraray). Human fetal liver cells or *BMI1*-transfected transformed cells were plated so that the number of cells per microcavity was five, and cultured (7.5x10⁴ cells/well of a 24-well plate). Medium exchange was performed 24 hr after plating; thereafter, medium was changed once in one or two days. At the time of medium exchange, addition of EGF and HGF was also carried out. The medium was of the same composition as in two-dimensional culture.

### Results and Discussion

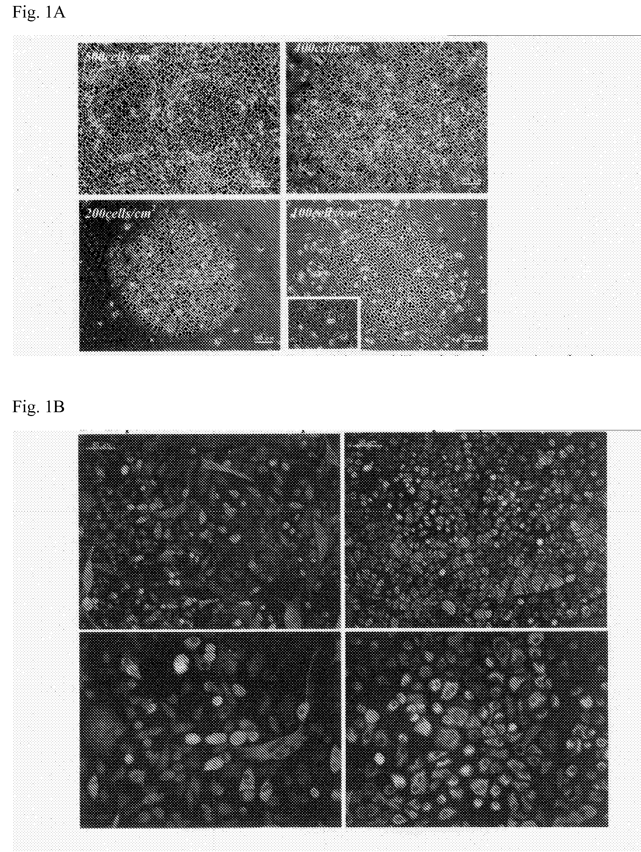

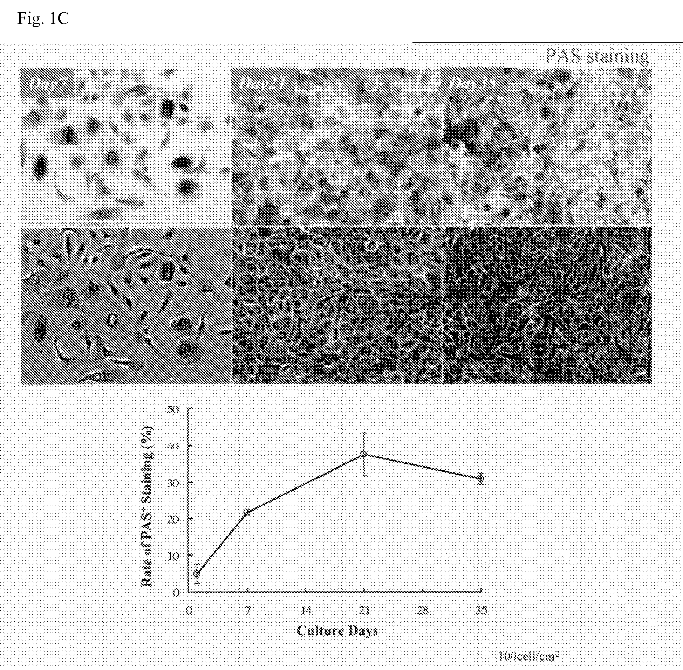

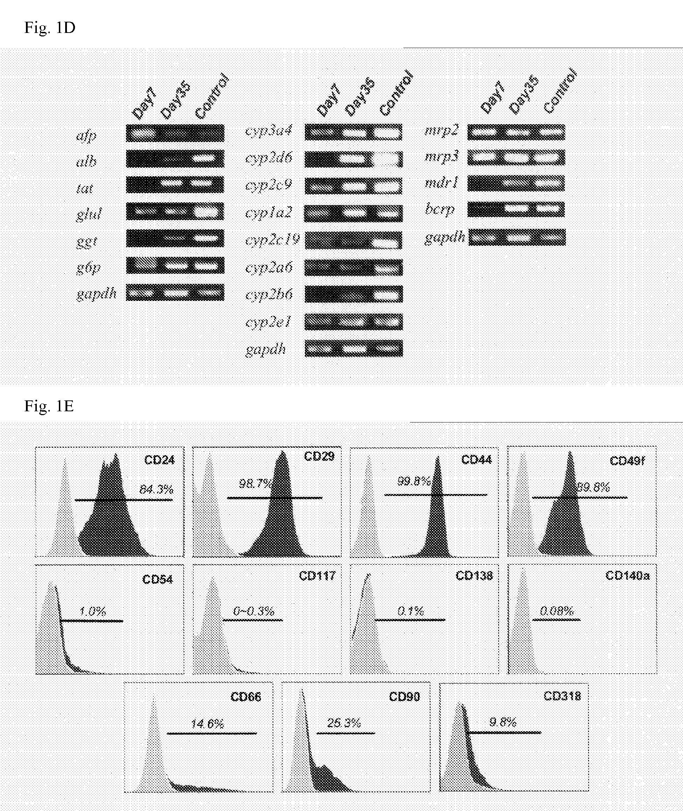

### Profiling of Human Primary Fetal Liver Cells (Fig. 1)

### A. Clonal Colony-Forming Efficiency of Human Primary Fetal Liver Cells in Low Density Culture (Fig. 1A)

As one of the most important indicators in identifying stem cells, the clonal colony-forming efficiency of the liver cells should be examined. The present inventors cultured human fetal liver cells at fetal week 14-18 at different plating densities (500, 400, 200 and 100 cells/cm²) using the low density culture method which is widely used for assessing clonal colony-forming efficiency. As a result, clonal colony formation was confirmed at plating densities of 200 cells/cm² and 100 cells/cm². Further, as shown in the inserted box, these colonies had a typical hepatocytic morphology.

### B. Bi-Potential Differentiation Capability of Human Primary Fetal Liver Cells In Vitro (Fig. 1B)

In order to identify human primary liver cells *in vitro,* the present inventors carried out immunocytochemical assays with the hepatocytic lineage marker albumin, the cholangiocytic lineage marker cytokeratin 19 and the undifferentiated liver cell (hepatoblast) marker AFP. As a result, the existence of cells that co-express two cell lineage markers (i.e., have bi-potential differentiation capability) was confirmed. From Fig. 1A and 1B, it is believed that the human primary liver cells the inventors used have the properties of stem cells. Therefore, the inventors judged that these liver cells are suitable for isolation of stem cells at higher enrichment and decided to use them in the present study.

### C. Differentiation Capability of Human Primary Fetal Liver Cells In Vitro (Glycogen Storage Capacity) (Fig. 1C)

In addition to the clonal colony-forming efficiency described above, differentiation capability is another important indicator to assess the potential as stem cell. The inventors carried out PAS staining on primary fetal liver cells at day 7, day 21 and day 35 of culture. The results revealed that glycogen storage increases in proportion to the number of days of culture. From this fact, it was proved that primary fetal liver cells differentiate from undifferentiated liver cells to functional liver cells. The upper panels of Fig. 1C show bright field images and the lower panels show phase contrast images.

### D. Differentiation Capability of Human Primary Fetal Liver Cells In Vitro (Gene Expression) (Fig. 1D)

In addition to expression analyses at the protein level in liver cells, expression analyses at the RNA level were also performed in the present study. Gene expression analyses were performed on liver function markers (5 types indicated at the left: AFP, ALB, TAT, GLUL, GGT, G6P), cytochrome P450 (8 types indicated in the center: CYP3A4, CYP2D6, CYP2C9, CYP1A2, CYP2C 19, CYP2A6, CYP2B6, CYP2E1) and transporters (4 types indicated at the right: MRP2, MRP3, MDR1, BCRP). The results revealed that almost all of these genes expressed in adult liver tissue are also expressed in the primary fetal liver cells induced for differentiation. It was also confirmed that their expression levels increase in proportion to the number of days of culture for induction of differentiation. As a positive control, human adult liver tissue was used.

From these results, the present inventors judged that the primary fetal liver cells obtained from ACBRI are suitable for isolation of stem cells at higher enrichment, and decided to use those cells in the present study.

### E. Surface Antigen Profiling on Human Primary Fetal Liver Cells by Flow Cytometry (Fig. 1E)

Profiling of the surface antigens of human primary fetal liver cells was performed using flow cytometry and fluorochrome-conjugated monoclonal antibodies.

As shown in the upper row of Fig. 1E, CD24, CD29, CD44 and CD49f were expressed in 80% or more of the primary fetal liver cells. As shown in the center row, positive cell ratios of CD54, CD 117, CD138 and CD 140a were less than 1%. As shown in the lower row, positive cell ratios of CD66, CD90 and CD318 were moderate.

Given the expressions of surface antigens that provide these extremely positive or negative cell ratios, it is operationally difficult to carry out cell separation using such expressions as an indicator, so the present inventors decided that to isolate and identify hepatic stem cells in the present study, the expressions of surface antigens that were verified to show moderately positive cell ratios in profiling should be used as an indicator for separation.

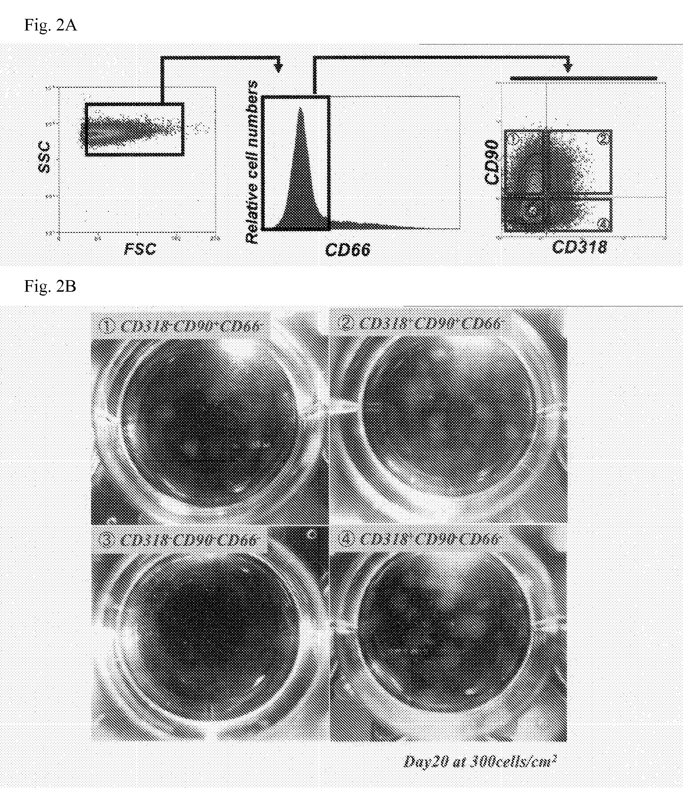

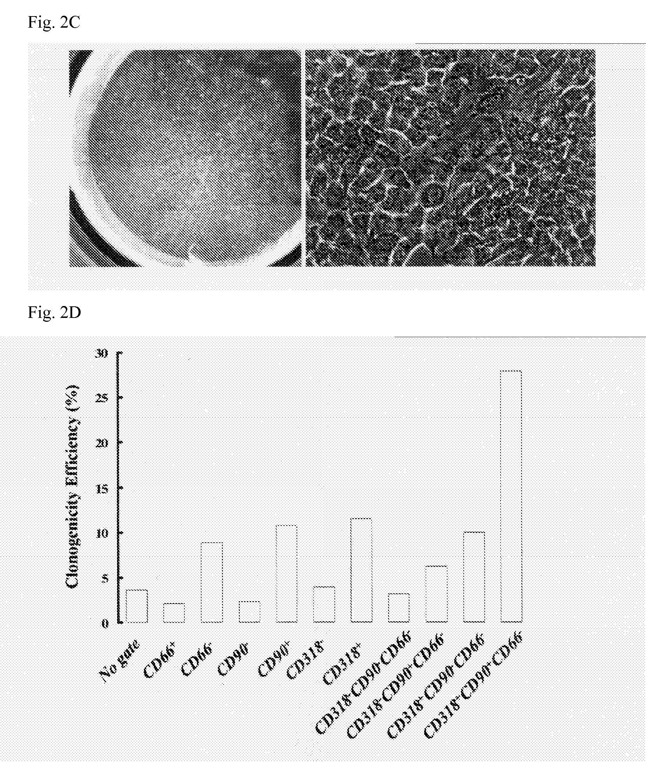

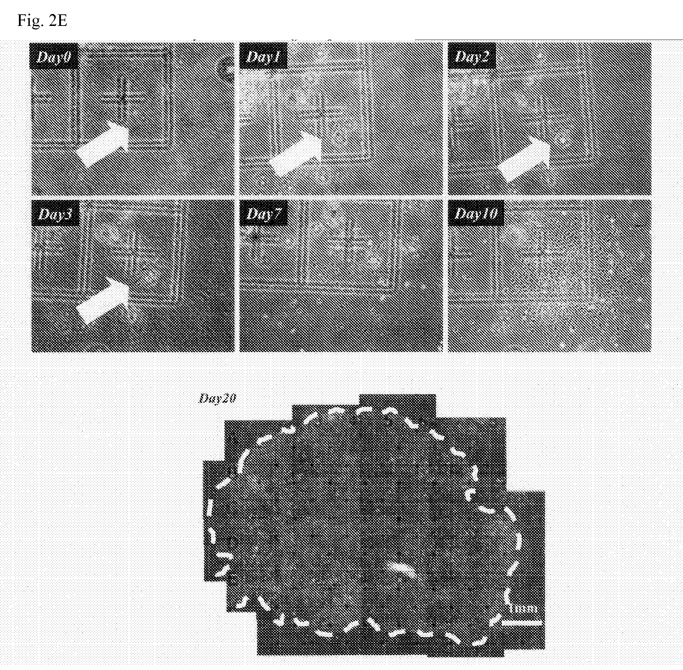

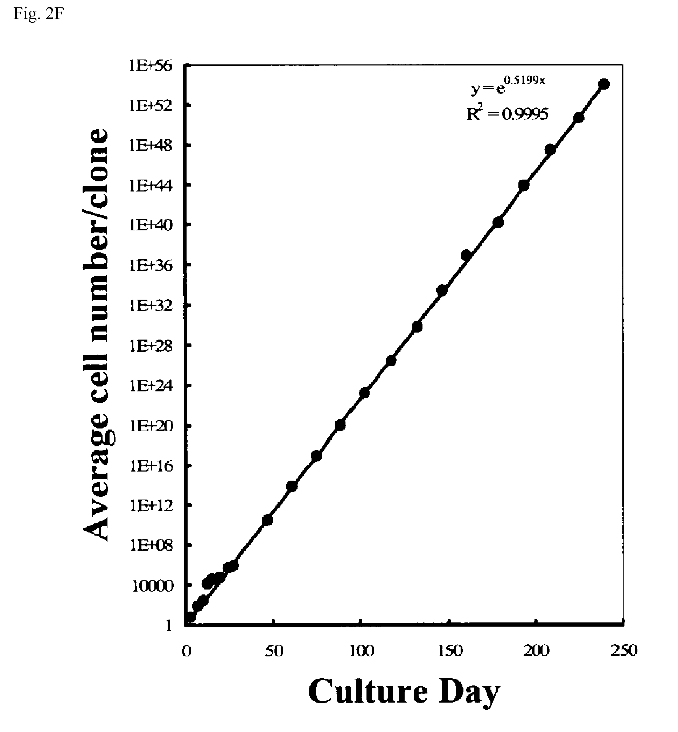

### Isolation and Characterization of Human Hepatic Stem Cells (Fig. 2)

### A. Isolation of Human Hepatic Stem Cells by Flow Cytometry (Fig. 2A)

This Figure shows a flow chart of cell sorting by flow cytometry.

First, cells were passed through the gate of FSC (Forward Scatter) and SSC (Side Scatter) to remove debris and fragments. Then, PI negative cell (viable cell) fraction was sorted out. This fraction was further fractionated using the expressions of CD66, CD90 and CD318 as an indicator. The inventors performed assessment of clonal colony-forming efficiency of a single cell for each of the positive and negative cell fractions corresponding to the expression of a single marker, as well as cell fractions No. 1 to No. 4 shown in the right panel. As a result, the present inventors found a cell population with high proliferative activity in CD318⁺CD90⁺CD66⁻ cell fraction. (Quantitative results are shown in Fig. 2D.)

### B. Phenotypes of Clonal Colonies in Individual Cell Fractions (Fig. 2B)

Cell sorting was performed using various monoclonal antibodies in varied combinations. Finally, a cell population with high clonal colony-forming efficiency was found in CD318⁺CD90⁺CD66⁻ cell fraction. Fig. 2B shows clonal colonies at day 20 of culture as fixed with 2% PFA. Clonal colonies derived from CD318⁺CD90⁺CD66⁻ cell fraction are cleanly seen in the right upper panel. The number of colony forming units (H-CFU-C) used as an indicator for assessing hepatic stem cells was the largest in CD318⁺CD90⁺CD66⁻ fraction-derived cells.

### C. Hepatic Stem Cell-Derived Clonal Colony (Fig. 2C)

In order to observe the clonal colony formation and pluripotency of hepatic stem cells, single cell sorting was performed in 96-well plates. At day 18 of culture, a clonal colony was formed as shown in Fig. 2C and it was confirmed that a single cell had expanded to 1.5 x 10⁴ cells. These cells had a typical morphology of hepatic cells and retained high proliferative activity throughout the culture period.

### D. A Single Cell-Derived Clonal Colony-Forming Efficiency In Vitro (Quantitative Analysis) (Fig. 2D)

For each of the cell fractions indicated in Fig. 2D, single cell-derived clonal colony-forming efficiency was assessed. The assessment was performed with type IV collagen-coated 96-well plates, and those wells showing colonies composed of more than 500 liver epithelial cells after three-week culture were counted. As a result, higher clonal colony-forming efficiency was confirmed in CD318⁺CD90⁺CD66⁻ cells than in other cell fractions. It was shown that hepatic stem cells were concentrated about 7.8 times more than non-fractionated cells.

### E. Tracking Observation of Hepatic Stem Cells In Vitro (Fig. 2E)

A single hepatic stem cell isolated by flow cytometry was monitored from day 1 through day 20 of culture. Hepatic stem cells showing vigorous proliferative activity *in vitro* are capable of forming a gigantic colony from a single cell. Fig. 2E shows only the image of a representative colony and single cell-derived gigantic colonies like the one shown in this Figure were observed with high frequency.

### F. Expansion Curve of Hepatic Stem Cells In Vitro (Fig. 2F)

In order to assess the proliferative activity of hepatic stem cells *in vitro,* cell culture from a single hepatic stem cell has now been continued for 8 months or longer. This cell still retains a typical morphology of liver cells and is showing stable proliferative activity.

Therefore, it can be concluded that CD318⁺CD90⁺CD66⁻ cells showing high proliferative capacity *in vitro* have properties of stem cells.

One of the human hepatic stem cell clones with the phenotypes of CD318⁺CD90⁺CD66⁻ was designated LSC-E2 and deposited at the International Patent Organism Depository, National Institute of Advanced Industrial Science and Technology (accession number: FERM BP-11108; date of accession: April 3, 2008).

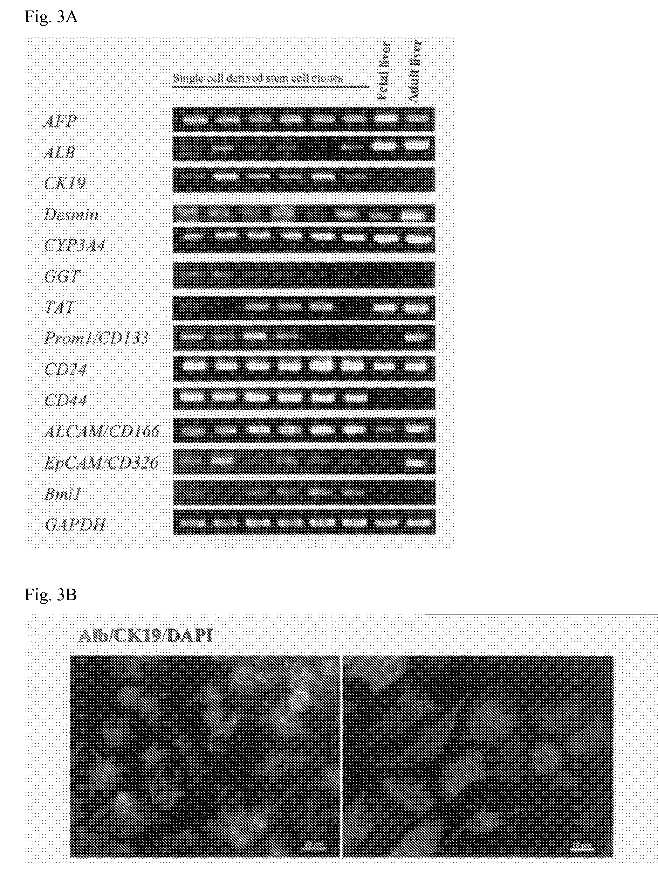

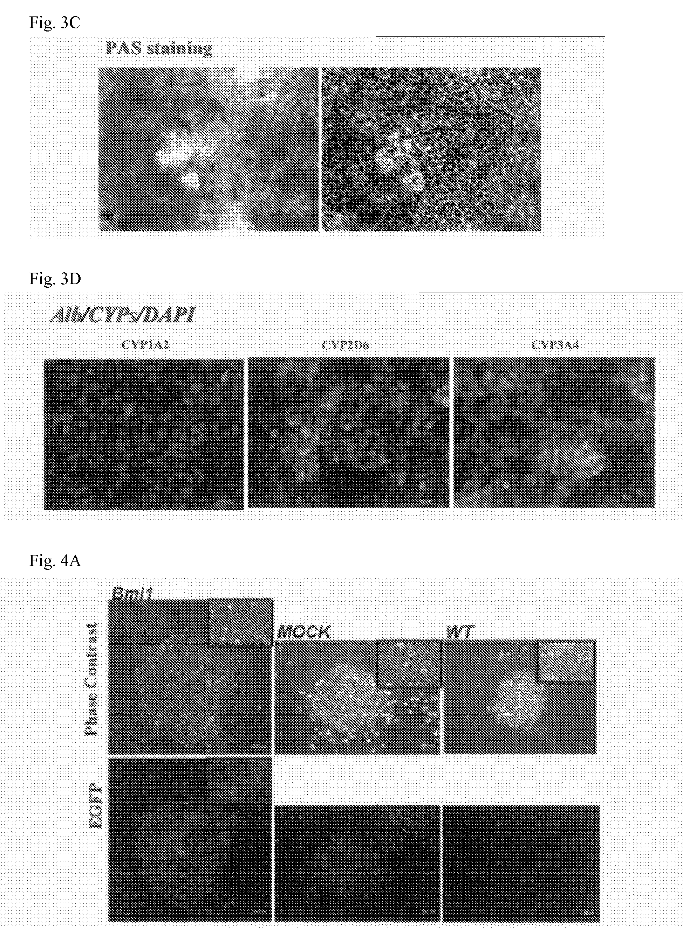

### Pluripotency of Human Hepatic Stem Cells (Fig. 3)

### A. Gene Expression Analyses in Hepatic Stem Cell-Derived Clonal Colonies (Fig. 3A)

Gene expression analyses were performed in order to prove that the isolated hepatic stem cells have not only proliferative capacity but also pluripotency. As shown in Fig. 3A, hepatocytic lineage markers (*AFP,* albumin), cholangiocytic lineage marker (cytokeratin 19), functional hepatocyte markers (*GGT, TAT, CYP3A4*) already confirmed to be expressed in mature liver cells, stem cell-associated genes (*BMI1, PROM1, CD24, CD44, ALCAM, EPCAM*) and other gene (*Desmin*) were confirmed to be expressed in single hepatic stem cell-derived clonal colonies.

### B. Expression Analyses of Hepatocyte and Cholangiocyte Markers in Hepatic Stem Cell-Derived Clonal Colonies (Fig. 3B)

A single hepatic stem cell was cultured in a 96-well plate, followed by immunocytochemical assays on albumin and cytokeratin 19. Fig. 3B confirms the expression of the hepatocytic lineage marker albumin (green) and the cholangiocytic lineage marker cytokeratin 19 (red). It is suggested that those cells which are positive for both albumin and cytokeratin 19 have bi-potent differentiation capability toward hepatocytic lineage and cholangiocytic lineage.

### C. Glycogen Storage Capacity of Hepatic Stem Cells (Fig. 3C)

PAS staining images of hepatic stem cells cultured for as long as 80 days are shown. The right panel shows a bright field image and the left panel a phase contrast image. Since PAS stain positive cell means that it has glycogen storage capacity, hepatic stem cells are believed to have differentiated as a result of long-term culture.

### D. Long-Term Culture of Hepatic Stem Cells and Expression of CYP450 (Fig. 3D)

The present inventors proved the differentiation capability of hepatic stem cell-derived clonal colonies after long-term culture for three months. The hepatic stem cell-derived clonal colonies expressed such markers as cytochrome P450 1A2, 2D6 and 3A4 that suggest differentiation capability like albumin. Since cells that are positive for both CYP 3A4 and albumin were found in the colonies, it is believed that isolated hepatic stem cells have differentiation capability and that functional liver cells can be maintained in the culture system used in the present study.

Therefore, it can be concluded that the hepatic stem cells isolated in the present study have pluripotency and can be induced to differentiate into functional liver cells through long-term culture.

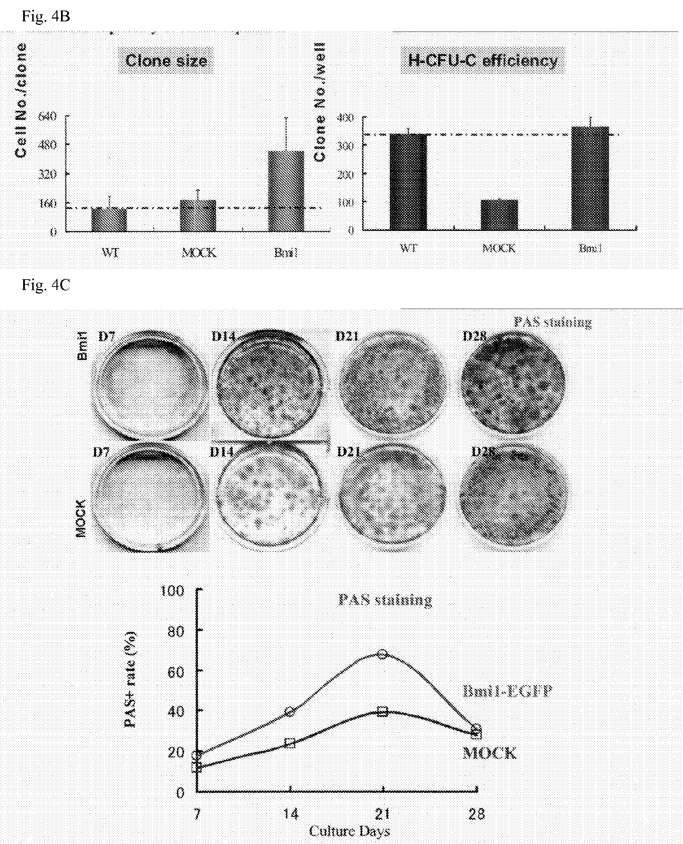

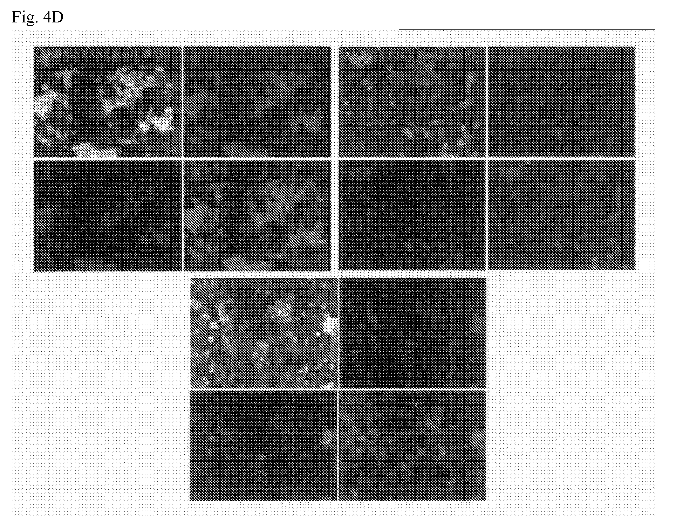

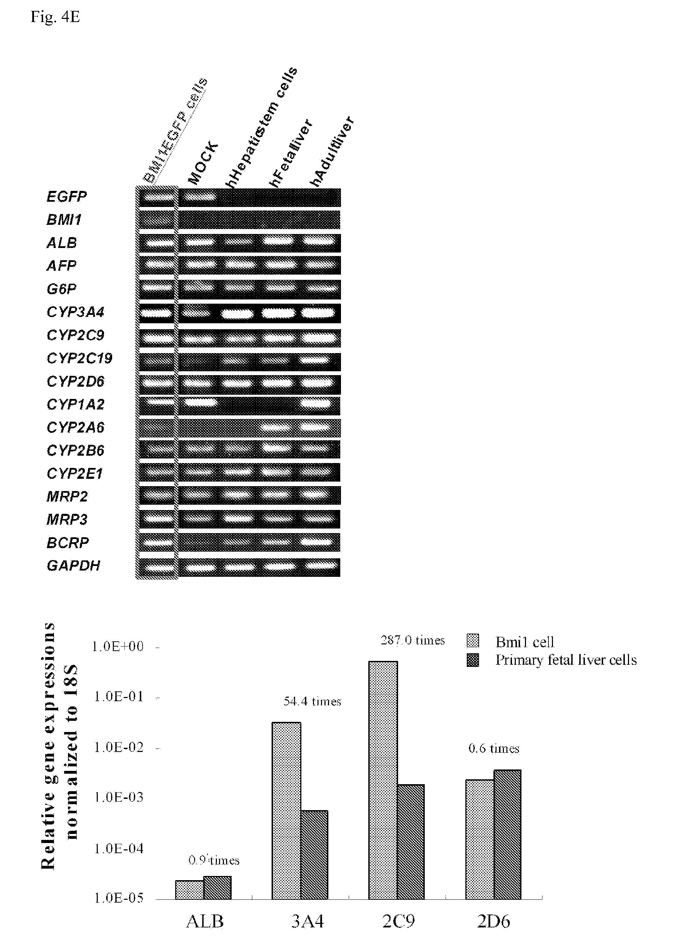

### Clonogenicity and Expression Analysis of Cytochrome P450 in BMI1-Transfected Human Hepatic Stem Cells (Fig. 4)

### A. Phenotype in BMI1 Overexpressing Hepatic Stem Cells

Introduction of *BMI1* gene into hepatic stem cells was confirmed with GFP fluorescence used as an indicator. No morphological difference was observed between *BMI1* overexpressing cells, mock and wild-type (WT) cells. However, *BMI1* overexpressing cell-derived colonies were larger than control cell-derived colonies.

### B. Clonal Colony-Forming Efficiency Enhanced by BMI1 Gene Introduction (Fig. 4B)

When the cell numbers in *BMI1* overexpressing cell-derived clonal colonies were compared to the cell numbers in mock- or WT-derived colonies, about two-fold increase was observed on average. Despite the introduction of *BMI1* gene into hepatic stem cells, no change was observed between those stem cells and WT as regards the number of H-CFU-C. On the other hand, the number of H-CFU-C in those stem cells was three times as large as that number in mock. These results made it clear that introduction of *BMI1* gene into hepatic stem cells enhances their proliferative capability but does not affect the frequency (H-CFU-C) of hepatic stem cells.

### C. Differentiation Capability of BMI1 Overexpressing Human Hepatic Stem Cells (Glycogen Storage Capacity) (Fig. 4C)

Since it is impossible to assess the properties of *BMI1* overexpressing cells by phenotype alone, their differentiation capability was analyzed using the expression of hepatocytic lineage markers and hepatic function markers as an indicator. Fig. 4C compares glycogen storage capacities in *BMI1* overexpressing cells and mock using PAS staining. As a result, *BMI1* overexpressing cells showed higher glycogen storage capacity than mock. Further, more than half of the *BMI1* overexpressing cells were found to be PAS positive at day 21 of culture.

### D. Cytochrome P450 Expression in BMI1 Overexpressing Human Hepatic Stem Cells (Immunocytochemistry) (Fig. 4D)

Immunocytochemical assays confirmed the expression of albumin and CYP3A4, 2C9 and 2D6 in *BMI1* overexpressing cells. Those cells which were positive for both albumin and cytochrome P450 produced very vivid stain images.

### E. Gene Expression Analyses in BMI1 Overexpressing Human Hepatic Stem Cells (Fig. 4E)

Expression of albumin, *AFP, G6P* and cytochrome P450 genes was confirmed by RT-PCR and Taqman real time PCR. From the results of RT-PCR shown in Fig. 4E, it became clear that *BMI1* overexpressing cells were expressing more hepatocytic lineage marker genes and cytochrome P450 genes than mock and WT. Further, the results of Taqman real time PCR revealed a significant increase in the expression levels of CYP3A4 and 2C9 genes in *BMI1* overexpressing cells. No significant difference was observed in the expression levels of albumin and CYP2D6 genes.

In summary, compared to wild-type stem cells, *BMI1* overexpressing stem cells possess higher clonal expansion capability and strongly express liver function markers such as cytochrome P450.

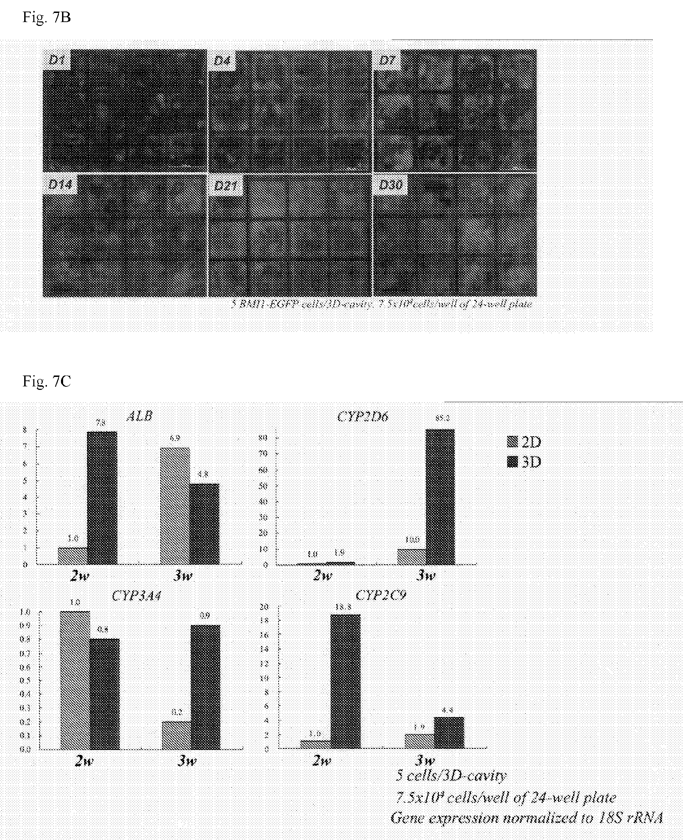

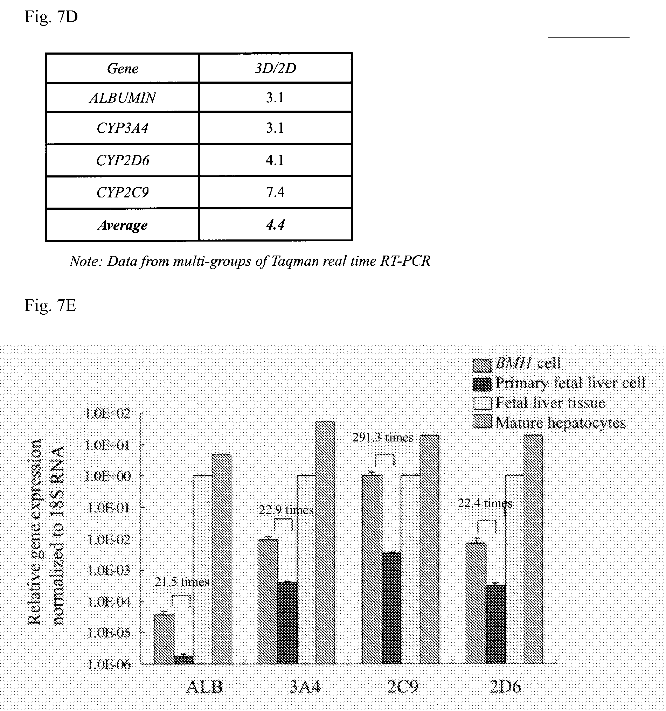

### Three-Dimensional Culture (Fig. 7)

### A. Three-Dimensional Culture System (Fig. 7A)

Bioreactor systems are gradually attracting attention as a three-dimensional culture system and are capable of culturing cells while maintaining the *in vivo* cell functions which were frequently lost in conventional two-dimensional culture. It is believed that this advantage is achieved because cell-to-cell interactions are enhanced in three-dimensional culture systems. The present inventors performed experiments using a three-dimensional culture system, or a simulated *in vivo* environment provided by Kuraray [a micropattemed film comprising numerous microcavities (L x W x D = 100 µm x 100 µm x 50 µm)].

The present inventors cultured cells at a plating density of 5 cells/cavity and confirmed cell adhesion and proliferation. Thus, it was demonstrated that smooth culture is possible with the three-dimensional culture system. Cells initially adhered to the bottom of the film, and with the passage of time, they gradually extended to other microcavities and formed a cellular aggregate at the center of each microcavity.

### B. Three-Dimensional Culture of BMI1-Transfected Transformed Cell (Fig. 7B)

Human hepatic stem cell fractions were sorted by flow cytometry, followed by overexpression of *BMI1* with a retrovirus vector (hereinafter, the thus prepared cell is called "*BMI1*-transfected transformed cell"). Since the expression of BMI1 can be confirmed using the expression of a reporter gene FGP as an indicator, *BMI1*-transfected transformed cell emits green fluorescence as shown in Fig. 7B. *BMI1*-transfected transformed cell indicated fairly good proliferative activity compared to those cells which did not undergo *BMI1* introduction. Although *BMI1*-transfected transformed cell showed a tendency to form multilayers, no big difference was observed in the localization of albumin and CYP3A4 and in glycogen storage capacity.

### C. Gene Expression Analyses on Human Hepatic Stem Cells in Two-Dimensional and Three-Dimensional Culture Systems (Fig. 7C)

Expressions of hepatocytic lineage markers and CYP450 genes were observed. Then, quantitative analyses of gene expressions were performed for each of the culture periods in two-dimensional and three-dimensional culture systems. The results revealed that the expression level of albumin gene in three-dimensional culture system decreased with the passage of time, but on the average, it exceeded the expression level of albumin gene in two-dimensional culture system. While the expression level of CYP3A4 gene decreased greatly in two-dimensional culture system with the passage of time, it retained a stable level in three-dimensional culture system and, on the average, exceeded the corresponding level in two-dimensional culture system. Further, the expression levels of CYP2D6 and CYP2C9 genes were higher in three-dimensional culture system than in two-dimensional culture system for both culture periods. As far as CYP2D6 gene is concerned, the expression level in three-dimensional culture system far exceeded the expression level in two-dimensional culture system. The expression of CYP2C9 gene achieved a high level by two-week culture in three-dimensional culture system. Although the expression level decreased with the passage of time, the expression still maintained a high level compared to the corresponding expression level in two-dimensional culture system. Therefore, it can be concluded that three-dimensional culture system contributes not only to the differentiation of human hepatic stem cells into liver cells that express drug-metabolizing enzymes, etc. but also to the maintenance of the functions of differentiated stem cells.

### D. Enhanced Gene Expression in Three-Dimensional Culture System (Fig. 7D)

The numerical values given in Fig. 7D are relative values obtained as described below. Briefly, the expression levels of individual genes in several samples obtained with two-dimensional and three-dimensional culture systems were determined by real time PCR; then, the expression level in three-dimensional culture system was divided by the corresponding expression level in two-dimensional culture system. These results clearly show that the expression levels of hepatocytic lineage marker and liver function marker genes are increased in three-dimensional culture system. The expression levels of individual genes in three-dimensional culture system are more than three times greater than the corresponding levels in two-dimensional culture system, showing an increase by a factor of 4.4 on average. With respect to albumin and CYP3A4, the values obtained were the same. Therefore, it is believed that three-dimensional culture system contributes to an enhancement of the expression of liver cell function genes including drug-metabolizing enzyme genes.

### E. Gene Expression Analysis of BMI1-Transfected Transformed Cell in Three-Dimensional Culture System (Fig. 7E)

Gene expression analysis was performed on *BMI1*-transfected transformed cell cultured three-dimensionally, human primary fetal liver cells, adult-derived human liver cells and human liver tissue using real time PCR. As a result, expression of drug-metabolizing enzymes equivalent to the expression in human primary fetal liver cells was confirmed in *BMI1*-transfected transformed cell cultured three-dimensionally. The expression levels of drug-metabolizing enzymes in *BMI1*-transfected transformed cell cultured three-dimensionally were confirmed to be close to the corresponding expression levels in human liver tissue and adult-derived human liver cells. This shows the efficacy of drug evaluation tests using *BMI1*-transfected transformed cell cultured three-dimensionally.

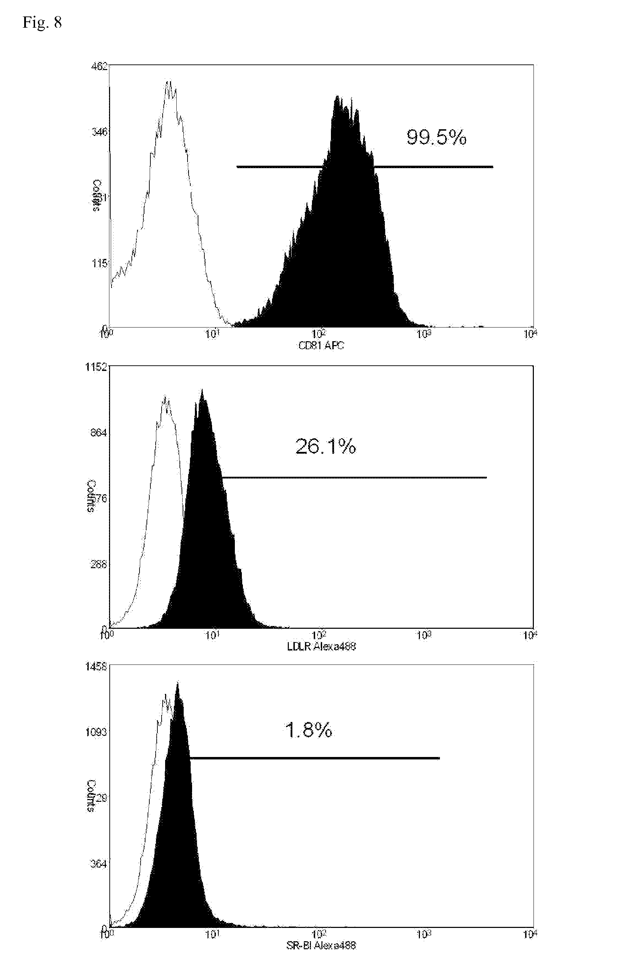

### Expression Analysis of HCV Receptors in Hepatic Stem Cells (Table 1, Fig. 8)

The most important point in HCV researches is to develop a highly reproducible and efficient culture system. Recently, several candidates for HCV receptors have been reported. For example, CD81 tetraspanin, scavenger receptor BI (SR-BI) and low-density lipoprotein (LDL) receptors may be enumerated. DNA microarray analyses of human primary fetal liver cells and isolated human hepatic stem cells using Affymetrix GeneChip Human Genome U133 Plus 2.0 Array and GeneSpring GX9.0 analysis software revealed that the expression level of HCV receptor CD81 in hepatic stem cells was significantly increased (Table 1). Further, flow cytometric analyses detected the expression of HCV receptors CD81 and LDLR (Fig. 8). Three-dimensional culture system combined with the above-described cell is applicable to HCV researches.

**Table 1**

| DNA Microarray | | |
|---|---|---|
| | Primary fetal liver cells | Hepatic stem cells |
| CD81 | 4605.6 | 8329.2 |
| SR-B1 | 740.6 | 615.2 |
| LDLR | 964.9 | 1115 |

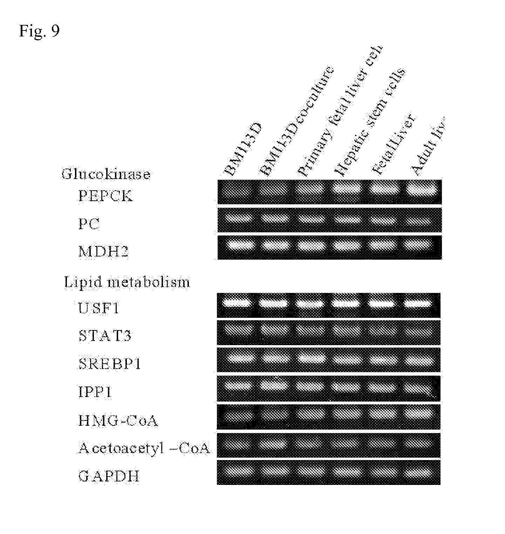

### Analyses of Lipid Metabolism- and Glucogenesis-Associated Genes in Human Liver Cells Induced to Differentiate from Hepatic Stem Cells (Fig. 9)

Gene expression analyses were performed by semi-quantitative PCR on *BMI1-*transfected transformed cells cultured three-dimensionally, co-culture of this transformed cell and vascular endothelial cells, as well as hepatic stem cells, primary fetal liver cells, and human liver tissue as a control. Table 2 shows the primer sequences used. As a result, expression of lipid metabolism- and glucogenesis-associated genes equivalent to the expression in human liver tissue was confirmed in *BMI1*-transfected transformed cell cultured three-dimensionally (Fig. 9). Further, the expression levels of lipid metabolism genes and glucogenesis-associated genes in *BMI1*-transfected transformed cell cultured three-dimensionally were confirmed to be close to the corresponding expression levels in human liver tissue (Fig. 9). From these results, applicability of *BMI1*-transfected transformed cell cultured three-dimensionally to the following has been suggested: elucidation of the mechanism of fatty liver development; evaluation of lipid metabolism regulatory agent; development of lipids-to-glucogenesis promoting agent; elucidation of the relationship between glucogenesis and diabetes; and evaluation tests such as metabolome analysis on cell metabolite during culture process.

Samples:
1) BMI1-3D: *BMI1*-transfected transformed cell cultured in three-dimensional culture system.
2) BMI1-3D co-culture: Culture obtained by co-culturing the above-described cell and human vascular endothelial cells in three-dimensional culture system.
3) Primary fetal liver cells: Primary human fetal liver cells.
4) Hepatic stem cells: Hepatic stem cells isolated from primary fetal liver cells.
5) Fetal Liver: Human fetal liver total RNA from Clontech, a Takara Bio Company.
6) Adult liver: Human liver total RNA from Clontech, a Takara Bio Company.

Lipid metabolism-associated genes:
1) upstream stimulatory factors 1(USF1)
2) sterol response element binding protein (SREBP-1)
3) acetoacetyl-CoA
4) Isopentenyl pyrophosphate 1 (IPP1)
4) HMG-CoA (3-hydroxy-3-methylglutaryl-coenzyme A)
5) STAT3 (signal transducer and activator of transcription 3)

Glucogenesis-associated genes
1) pyruvate carboxylase (PC)
2) malate dehydrogenase 2 (MDH2)
3) phosphoenolpyruvate carboxykinase (PEPCK)

**Table 2**

| | | | |
|---|---|---|---|
| PEPCK | NM_004563. | 5'-gcacatcccaactctcgatt-3' | (SEQ ID NO: 57) |
| | | 5'-gagggagaacagctgagtgg-3' | (SEQ ID NO: 58) |
| | | | |
| PC | NM_001040716. | 5'-tcctgcagaatgtgctcaac-3' | (SEQ ID NO: 59) |
| | | 5'-cagcatctggaaagggatgt-3' | (SEQ ID NO: 60) |
| | | | |
| MDH2 | NM_005918 | 5'-gcagccactttcacttctcc-3' | (SEQ ID NO: 61) |
| | | 5'-cattggtgttgaacaggtcg-3' | (SEQ ID NO: 62) |
| | | | |
| STAT3 | NM_003150 | 5'-aactcttgggacctggtgtg-3' | (SEQ ID NO: 63) |
| | | 5'-taggcgcctcagtcgtatct-3' | (SEQ ID NO: 64) |
| | | | |
| USF1 | NM_007122 | 5'-accccaacgtcaagtacgtc-3' | (SEQ ID NO: 65) |
| | | 5'-acgacctctaatccgtggtg-3' | (SEQ ID NO: 66) |
| | | | |
| SREBF1 | NM_001005291 | 5'-cggagaagctgcctatcaac-3' | (SEQ ID NO: 67) |
| | | 5'-ggtcagtgtgtcctccacct-3' | (SEQ ID NO: 68) |
| | | | |
| Acetoacetyl-CoA | | NM_023928 | |
| | | 5'-aaaggcagtcggctcaacta-3' | (SEQ ID NO: 69) |
| | | 5'-gtccagcacaccattcacac-3' | (SEQ ID NO: 70) |
| | | | |
| IPP1 | NM_004508. | 5'-ctcgacaagcaacaggttca-3' | (SEQ ID NO: 71) |
| | | 5'-ctcagctttcagccgtctct-3' | (SEQ ID NO: 72) |
| | | | |
| HMG-CoA | | NM_000191. | |
| | | 5'-cctggcatcaactacccagt-3' | (SEQ ID NO: 73) |
| | | 5'-ccagggagatctcgtagcag-3' | (SEQ ID NO: 74) |

### Expression Analysis of CD 13 in Human Hepatic Stem Cells

CD13 is one of mesenchymal stem cell markers. DNA microarray analysis using Affymetrix GeneChip Human Genome U133 Plus 2.0 Array and GeneSpring GX9.0 analysis software revealed that the expression level of CD 13 in isolated hepatic stem cells was 2.7 times higher than the corresponding expression level in primary fetal liver cells (4001.1/1457.7).

All publications, patents and patent applications cited herein are incorporated herein by reference in their entirety.

### INDUSTRIAL APPLICABILITY

The human hepatic stem cell isolated according to the present invention is capable of contributing greatly to regenerative medicine such as artificial liver or cell transplantation. Human liver cells induced to differentiate from the isolated human hepatic stem cell may be used in drug development researches such as analysis of *in vitro* drug-metabolizing ability. In particular, *BMI1*-transfected human hepatic stem cells may be handled as a cell strain in cell operations and human liver cells induced to differentiate from them have extremely high utility in drug development industry. Further, these cells may be used as human model cells in experiments on HCV infection and/or inhibition of HCV replication, glucose metabolism experiments, lipid metabolism experiments, and so forth.

### SEQUENCE LISTING FREE TEXT

<SEQ ID NOS: 1 and 2>
   SEQ ID NOS: 1 and 2 show the sequences of liver cell specific primers for albumin.
<SEQ ID NOS: 3 and 4>
   SEQ ID NOS: 3 and 4 show the sequences of liver cell specific primers for *AFP.*
<SEQ ID NOS: 5 and 6>
   SEQ ID NOS: 5 and 6 show the sequences of liver cell specific primers for *GP6.*
<SEQ ID NOS: 7 and 8>
   SEQ ID NOS: 7 and 8 show the sequences of liver cell specific primers for *CYP3A4.*
<SEQ ID NOS: 9 and 10>
   SEQ ID NOS: 9 and 10 show the sequences of liver cell specific primers for *CYP2C9.*
<SEQ ID NOS: 11 and 12>
   SEQ ID NOS: 11 and 12 show the sequences of liver cell specific primers for *CYP2C19.*
<SEQ ID NOS: 13 and 14>
   SEQ ID NOS: 13 and 14 show the sequences of liver cell specific primers for *CYP2D6.*
<SEQ ID NOS: 15 and 16>
   SEQ ID NOS: 15 and 16 show the sequences of liver cell specific primers for *CYP1A2.*
<SEQ ID NOS: 17 and 18>
   SEQ ID NOS: 17 and 18 show the sequences of liver cell specific primers for *CYP2B6.*
<SEQ ID NOS: 19 and 20>
   SEQ ID NOS: 19 and 20 show the sequences of liver cell specific primers for *CYP2E1*.
<SEQ ID NOS: 21 and 22>
   SEQ ID NOS: 21 and 22 show the sequences of liver cell specific primers for *GAPDH.*
<SEQ ID NOS: 23 and 24>
   SEQ ID NOS: 23 and 24 show the sequences of liver cell specific primers for *EGFP.*
<SEQ ID NOS: 25 and 26>
   SEQ ID NOS: 25 and 26 show the sequences of liver cell specific primers for *BMI1.*
<SEQ ID NOS: 27 and 28>
   SEQ ID NOS: 27 and 28 show the sequences of liver cell specific primers for *TAT.*
<SEQ ID NOS: 29 and 30>
   SEQ ID NOS: 29 and 30 show the sequences of liver cell specific primers for *GULU.*
<SEQ ID NOS: 31 and 32>
   SEQ ID NOS: 31 and 32 show the sequences of liver cell specific primers for *GGT.*
<SEQ ID NOS: 33 and 34>
   SEQ ID NOS: 33 and 34 show the sequences of specific primers for *Desmin.*
<SEQ ID NOS: 35 and 36>
   SEQ ID NOS: 35 and 36 show the sequences of liver cell specific primers for *CYP2A6.*
<SEQ ID NOS: 37 and 38>
   SEQ ID NOS: 37 and 38 show the sequences of liver cell specific primers for *MRP2.*
<SEQ ID NOS: 39 and 40>
   SEQ ID NOS: 39 and 40 show the sequences of liver cell specific primers for *MDR1.*
<SEQ ID NOS: 41 and 42>
   SEQ ID NOS: 41 and 42 show the sequences of liver cell specific primers for *MRP3.*
<SEQ ID NOS: 43 and 44>
   SEQ ID NOS: 43 and 44 show the sequences of liver cell specific primers for *BCRP.*
<SEQ ID NOS: 45 and 46>
   SEQ ID NOS: 45 and 46 show the sequences of specific primers for *PROM1.*
<SEQ ID NOS: 47 and 48>
   SEQ ID NOS: 47 and 48 show the sequences of specific primers for *CD24.*
<SEQ ID NOS: 49 and 50>
   SEQ ID NOS: 49 and 50 show the sequences of specific primers for *CD44.*
<SEQ ID NOS: 51 and 52>
   SEQ ID NOS: 51 and 52 show the sequences of specific primers for *ALCAM.*
<SEQ ID NOS: 53 and 54>
   SEQ ID NOS: 53 and 54 show the sequences of specific primers for *EPCAM.*
<SEQ ID NOS: 55 and 56>
   SEQ ID NOS: 55 and 56 show the nucleotide sequence of human *BMI1* used in the present study (CDS nucleotide sequence (981 bp)) and the amino acid sequence thereof (326 amino acids), respectively (GenBank accession number: NM_005180.5).
<SEQ ID NOS: 57 and 58>
   SEQ ID NOS: 57 and 58 show the sequences of specific primers for PEPCK.
<SEQ ID NOS: 59 and 60>
   SEQ ID NOS: 59 and 60 show the sequences of specific primers for PC.
<SEQ ID NOS: 61 and 62>
   SEQ ID NOS: 61 and 62 show the sequences of specific primers for MDH2.
<SEQ ID NOS: 63 and 64>
   SEQ ID NOS: 63 and 64 show the sequences of specific primers for STAT3.
<SEQ ID NOS: 65 and 66>
   SEQ ID NOS: 65 and 66 show the sequences of specific primers for USF1.
<SEQ ID NOS: 67 and 68>
   SEQ ID NOS: 67 and 68 show the sequences of specific primers for SREBF1.
<SEQ ID NOS: 69 and 70>
   SEQ ID NOS: 69 and 70 show the sequences of specific primers for Acetoacetyl-CoA.
<SEQ ID NOS: 71 and 72 >
   SEQ ID NOS: 71 and 72 show the sequences of specific primers for IPP 1.
<SEQ ID NOS: 73 and 74 >
   SEQ ID NOS: 73 and 74 show the sequences of specific primers for HMG-CoA.

## Claims

1. A human hepatic stem cell sorted based on the presence or absence of the expression of at least one marker selected from the group consisting of CD318, CD90, CD66 and CD 13.

2. The human hepatic stem cell according to claim 1, which has the phenotype of CD318⁺.

3. The human hepatic stem cell according to claim 1 or 2, which has the phenotype of CD90⁺.

4. The human hepatic stem cell according to any one of claims 1 to 3, which has the phenotype of CD66⁻.

5. The human hepatic stem cell according to claim 1, which has the phenotypes of CD318⁺CD90⁺CD66⁻.

6. The human hepatic stem cell according to claim 5, which is a cell clone LSC-E2 deposited at the International Patent Organism Depository, National Institute of Advanced Industrial Science and Technology, Japan under accession number FERM BP-11108.

7. The human hepatic stem cell according to any one of claims 1 to 6, which has the phenotype of CD 13⁺.

8. The human hepatic stem cell according to claim 1, which is a cell, or a progeny thereof, that has been obtained by proliferating into a cell population a human hepatic stem cell clone sorted based on the presence or absence of the expression of at least one marker selected from the group consisting of CD318, CD90, CD66 and CD 13, and then sorting cells from the cell population based on the presence or absence of the expression of at least one marker selected from the group consisting of CD318, CD90, CD66 and CD 13.

9. A transformed cell, wherein *BMI1* gene has been introduced into the human hepatic stem cell according to claim 1.

10. A method of preparing the human hepatic stem cell according to claim 1, which comprises sorting cells from a human liver cell population based on the presence or absence of the expression of at least one marker selected from the group consisting of CD318, CD90, CD66 and CD13.

11. The method according to claim 10, wherein the human liver cell population is a primary liver cell culture line isolated from a human liver.

12. The method according to claim 10, wherein after sorting cells from a human liver cell population based on the presence or absence of the expression of at least one marker selected from the group consisting of CD318, CD90, CD66 and CD13, the resultant cells are proliferated into a cell population and then cells are sorted again from this cell population based on the presence or absence of the expression of at least one marker selected from the group consisting of CD318, CD90, CD66 and CD13.

13. The method according to claim 12, wherein cell sorting and proliferation are repeated twice or more.

14. A method of preparing a liver cell which expresses drug-metabolizing enzymes and/or transporters at the protein level, the method of comprising inducing the differentiation of the human hepatic stem cell according to claim 1 and/or the transformed cell according to claim 9.

15. The method according to claim 14, wherein the differentiation of the human hepatic stem cell according to claim 1 and/or the transformed cell according to claim 9 is induced by treatment with an extracellular matrix and/or a growth factor.

16. The method according to claim 15, wherein the extracellular matrix and/or growth factor is at least one member of the group consisting of laminin, type I collagen, type IV collagen, fibronectin, metrigel, dexamethasone, DMSO, oncostatin M, insulin, HGF, EGF, TGFα, HB-EGF, VEGF and PDGF.

17. The method according to claim 14, wherein the differentiation of the human hepatic stem cell according to claim 1 and/or the transformed cell according to claim 9 is induced by three-dimensional culture.

18. A liver cell expressing drug-metabolizing enzymes and/or transporters at the protein level, which has been prepared by the method according to claim 14.

19. The liver cell according to claim 18, wherein the drug-metabolizing enzyme is at least one member of the group consisting of CYP3A4, CYP2C9, CYP2C19, CYP2D6, CYP2B6, CYP2E1 and CYP2A6.

20. The liver cell according to claim 18, wherein the transporter is at least one member of the group consisting of ABC2, ABCA6, ABCA8, MDR1, MDR3, BSEP, MRP1, MRP2, MRP5, MRP6, ABCG8, NTCP, PEPT1, OATP-C, OATP8, OATP-B, OATP-F, OCT1, OAT2, SLC22A18, CNT1, ENT1, MATE1, MRP3 and BCRP.

21. A method of evaluating the metabolism and/or transportation of a drug candidate compound using the liver cell according to claim 18.

22. A bioreactor comprising the human hepatic stem cell according to claim 1, the transformed cell according to claim 9 and/or a liver cell which has been induced to differentiate therefrom.

23. The bioreactor according to claim 22, which is for use as an artificial liver.

24. A human model cell for experiments on HCV infection and/or inhibition of HCV replication, comprising the human hepatic stem cell according to claim 1, the transformed cell according to claim 9 and/or a liver cell which has been induced to differentiate therefrom.

25. A human model cell for glucose metabolism experiments, comprising the human hepatic stem cell according to claim 1, the transformed cell according to claim 9 and/or a liver cell which has been induced to differentiate therefrom.

26. A human model cell for lipid metabolism experiments, comprising the human hepatic stem cell according to claim 1, the transformed cell according to claim 9 and/or a liver cell which has been induced to differentiate therefrom.

27. A method of using in regenerative medicine with the human hepatic stem cell according to claim 1, the transformed cell according to claim 9 and/or a liver cell which has been induced to differentiate therefrom.

28. The method according to claim 27, wherein the regenerative medicine is cell therapy.


SEQUENCE LISTING

<110>  Public University Corporation Yokohama City University

<120>  Human hepatic stem cells, and methods of preparation, differentiation and use of the same

<130>  FP-129PCT

<150>  JP P2008-127755
<151>  2008-05-14

<160>  74

<170>  PatentIn version 3.1

<210>  1
<211>  20
<212>  DNA
<213>  Artificial

<220>
<223>  hepatocyte-specific primer for albumin

<400>  1
tgttgattgc ctttgctcag                                                    20


<210>  2
<211>  20
<212>  DNA
<213>  Artificial

<220>
<223>  hepatocyte-specific primer for albumin

<400>  2
tggagactgg cacacttgag                                                    20


<210>  3
<211>  20
<212>  DNA
<213>  Artificial

<220>
<223>  hepatocyte-specific primer for AFP

<400>  3
agcttggtgg tggatgaaac                                                    20


<210>  4
<211>  20
<212>  DNA
<213>  Artificial

<220>
<223>  hepatocyte-specific primer for AFP

<400>  4
ccctcttcag caaagcagac                                                    20


<210>  5
<211>  20
<212>  DNA
<213>  Artificial

<220>
<223>  hepatocyte-specific primer for G6P

<400>  5
gtcaacacat tacctccagg                                                    20


<210>  6

<211>  20
<212>  DNA
<213>  Artificial

<220>
<223>  hepatocyte-specific primer for G6P

<400>  6
gagtagatgt gaccatcacg                                                    20


<210>  7
<211>  20
<212>  DNA
<213>  Artificial

<220>
<223>  hepatocyte-specific primer for CYP3A4

<400>  7
aacagcctgt gctggctatc                                                    20


<210>  8
<211>  20
<212>  DNA
<213>  Artificial

<220>
<223>  hepatocyte-specific primer for CYP3A4

<400>  8
gatcacatcc atgctgtagg                                                    20


<210>  9
<211>  20
<212>  DNA
<213>  Artificial

<220>
<223>  hepatocyte-specific primer for CYP2C9

<400>  9
gcaggaaaac ggatttgtgt                                                    20


<210>  10
<211>  20
<212>  DNA
<213>  Artificial

<220>
<223>  hepatocyte-specific primer for CYP2C9

<400>  10
caggccatct gctcttcttc                                                    20


<210>  11
<211>  20
<212>  DNA
<213>  Artificial

<220>
<223>  hepatocyte-specific primer for CYP2C19

<400>  11
cttcaccctg tgatcccact                                                    20


<210>  12
<211>  20
<212>  DNA
<213>  Artificial

<220>
<223>  hepatocyte-specific primer for CYP2C19

<400>  12
taaagtcccg agggttgttg                                                    20


<210>  13
<211>  20
<212>  DNA
<213>  Artificial

<220>
<223>  hepatocyte-specific primer for CYP1A2

<400>  13
aggaggagtc gggctttctg                                                    20


<210>  14
<211>  20
<212>  DNA
<213>  Artificial

<220>
<223>  hepatocyte-specific primer for CYP2D6

<400>  14
cttggccttc tccatctctg                                                    20


<210>  15
<211>  20
<212>  DNA
<213>  Artificial

<220>
<223>  hepatocyte-specific primer for CYP1A2

<400>  15
cagaatgccc tcaacacctt                                                    20


<210>  16
<211>  20
<212>  DNA
<213>  Artificial

<220>
<223>  hepatocyte-specific primer for CYP1A2

<400>  16
gctcctggac tgttttctgc                                                    20


<210>  17
<211>  20
<212>  DNA
<213>  Artificial

<220>
<223>  hepatocyte-specific primer for CYP2B6

<400>  17
aacctgcagg aaatcaatgc                                                    20


<210>  18
<211>  20
<212>  DNA
<213>  Artificial

<220>
<223>  hepatocyte-specific primer for CYP2B6

<400>  18

attttggctc ggtcatgaag                                                    20

<210>  19
<211>  20
<212>  DNA
<213>  Artificial

<220>
<223>  hepatocyte-specific primer for CYP2E1

<400>  19
acccgagaca ccattttcag                                                    20

<210>  20
<211>  20
<212>  DNA
<213>  Artificial

<220>
<223>  hepatocyte-specific primer for CYP2E1

<400>  20
agggtgtcct ccacacactc                                                    20

<210>  21
<211>  20
<212>  DNA
<213>  Artificial

<220>
<223>  hepatocyte-specific primer for GAPDH

<400>  21
ctctgctcct cctgttcgac                                                    20

<210>  22
<211>  20
<212>  DNA
<213>  Artificial

<220>
<223>  hepatocyte-specific primer for GAPDH

<400>  22
ttgattttgg agggatctcg                                                    20

<210>  23
<211>  20
<212>  DNA
<213>  Artificial

<220>
<223>  hepatocyte-specific primer for EGFP

<400>  23
ctacggcgtg cagtgcttca                                                    20

<210>  24
<211>  20
<212>  DNA
<213>  Artificial

<220>
<223>  hepatocyte-specific primer for EGFP

<400>  24
cgctgccgtc ctcgatgttg                                                    20

<210>  25

<211>  20
<212>  DNA
<213>  Artificial

<220>
<223>  hepatocyte-specific primer for BMI1

<400>  25
   aatccccacc tgatgtgtgt                                                    20

<210>  26
<211>  20
<212>  DNA
<213>  Artificial

<220>
<223>  hepatocyte-specific primer for BMI1

<400>  26
catttttgaa aagccctgga                                                     20

<210>  27
<211>  18
<212>  DNA
<213>  Artificial

<220>
<223>  hepatocyte-specific primer for TAT

<400>  27
ttctggcagt ggctgcac                                                       18

<210>  28
<211>  20
<212>  DNA
<213>  Artificial

<220>
<223>  hepatocyte-specific primer for TAT

<400>  28
cccatctcgg atctcattgc                                                     20

<210>  29
<211>  20
<212>  DNA
<213>  Artificial

<220>
<223>  hepatocyte-specific primer for GULU

<400>  29
aaggcatcaa gcaggtgtac                                                     20

<210>  30
<211>  20
<212>  DNA
<213>  Artificial

<220>
<223>  hepatocyte-specific primer for GULU

<400>  30
gctggttgct caccatgtcc                                                     20

<210>  31
<211>  20
<212>  DNA
<213>  Artificial

<220>
<223>  hepatocyte-specific primer for GGT

<400>  31
acaaccatgt gtacaccagg                                                    20


<210>  32
<211>  20
<212>  DNA
<213>  Artificial

<220>
<223>  hepatocyte-specific primer for GGT

<400>  32
ctggtgtgcc agctcatacg                                                    20


<210>  33
<211>  20
<212>  DNA
<213>  Artificial

<220>
<223>  hepatocyte-specific primer for Desmin

<400>  33
aagctgcagg aggagattca                                                    20


<210>  34
<211>  20
<212>  DNA
<213>  Artificial

<220>
<223>  hepatocyte-specific primer for Desmin

<400>  34
ggcagtgagg tctggcttag                                                    20


<210>  35
<211>  20
<212>  DNA
<213>  Artificial

<220>
<223>  hepatocyte-specific primer for CYP2A6

<400>  35
agaggaagag caaggggaag                                                    20


<210>  36
<211>  20
<212>  DNA
<213>  Artificial

<220>
<223>  hepatocyte-specific primer for CYP2A6

<400>  36
ctgaactcct cagcctggtc                                                    20


<210>  37
<211>  20
<212>  DNA
<213>  Artificial

<220>
<223>  hepatocyte-specific primer for MRP2

<400>  37

tgcttcctgg ggataatcag                                                    20

<210>  38
<211>  20
<212>  DNA
<213>  Artificial

<220>
<223>  hepatocyte-specific primer for MRP2

<400>  38
cacggataac tggcaaacct                                                    20

<210>  39
<211>  20
<212>  DNA
<213>  Artificial

<220>
<223>  hepatocyte-specific primer for MDR1

<400>  39
gctcctgact atgccaaagc                                                    20

<210>  40
<211>  20
<212>  DNA
<213>  Artificial

<220>
<223>  hepatocyte-specific primer for MDR1

<400>  40
tcttcacctc caggctcagt                                                    20

<210>  41
<211>  20
<212>  DNA
<213>  Artificial

<220>
<223>  hepatocyte-specific primer for MRP3

<400>  41
tgtgctaggt gatggacagg                                                    20

<210>  42
<211>  20
<212>  DNA
<213>  Artificial

<220>
<223>  hepatocyte-specific primer for MRP3

<400>  42
tgtcacctgc accttctctg                                                    20

<210>  43
<211>  20
<212>  DNA
<213>  Artificial

<220>
<223>  hepatocyte-specific primer for BCRP

<400>  43
ttttctgttc ccttgccatc                                                    20

<210>  44

<211>  20
<212>  DNA
<213>  Artificial

<220>
<223>  hepatocyte-specific primer for BCRP

<400>  44
cctggccctc tactctaccc                                                    20


<210>  45
<211>  20
<212>  DNA
<213>  Artificial

<220>
<223>  hepatocyte-specific primer for PROM1

<400>  45
tccaacaggg ctatcaatcc                                                    20


<210>  46
<211>  23
<212>  DNA
<213>  Artificial

<220>
<223>  hepatocyte-specific primer for PROM1

<400>  46
ccaaagacaa aggtaagaac cac                                                23


<210>  47
<211>  21
<212>  DNA
<213>  Artificial

<220>
<223>  hepatocyte-specific primer for CD24

<400>  47
cccatcttct aaacccaatc c                                                  21


<210>  48
<211>  20
<212>  DNA
<213>  Artificial

<220>
<223>  hepatocyte-specific primer for CD24

<400>  48
cattccacaa tcccatcctt                                                    20


<210>  49
<211>  20
<212>  DNA
<213>  Artificial

<220>
<223>  hepatocyte-specific primer for CD44

<400>  49
tttacagcct cagcagagca                                                    20


<210>  50
<211>  20
<212>  DNA
<213>  Artificial

<220>
<223>  hepatocyte-specific primer for CD44

<400>  50
cgatgctcag agctttctcc                                                    20


<210>  51
<211>  20
<212>  DNA
<213>  Artificial

<220>
<223>  hepatocyte-specific primer for ALCAM

<400>  51
cgtctgctct tctgcctctt                                                    20


<210>  52
<211>  20
<212>  DNA
<213>  Artificial

<220>
<223>  hepatocyte-specific primer for ALCAM

<400>  52
tggttgcttg aacaccttga                                                    20


<210>  53
<211>  20
<212>  DNA
<213>  Artificial

<220>
<223>  hepatocyte-specific primer for EPCAM

<400>  53
tgatcctgac tgcgatgaga                                                    20


<210>  54
<211>  20
<212>  DNA
<213>  Artificial

<220>
<223>  hepatocyte-specific primer for EPCAM

<400>  54
gcacaacaat tccagcaaca                                                    20


<210>  55
<211>  981
<212>  DNA
<213>  Homo sapiens

<400>  55
atgcatcgaa caacgagaat caagatcact gagctaaatc cccacctgat gtgtgtgctt      60

tgtggagggt acttcattga tgccacaacc ataatagaat gtctacattc cttctgtaaa     120

acgtgtattg ttcgttacct ggagaccagc aagtattgtc ctatttgtga tgtccaagtt     180

cacaagacca gaccactact gaatataagg tcagataaaa ctctccaaga tattgtatac     240

aaattagttc cagggctttt caaaaatgaa atgaagagaa gaagggattt ttatgcagct     300

catccttctg ctgatgctgc caatggctct aatgaagata gaggagaggt tgcagatgaa     360

gataagagaa ttataactga tgatgagata ataagcttat ccattgaatt ctttgaccag     420

aacagattgg atcggaaagt aaacaaagac aaagagaaat ctaaggagga ggtgaatgat     480

aaaagatact tacgatgccc agcagcaatg actgtgatgc acttaagaaa gtttctcaga 540
agtaaaatgg acatacctaa tactttccag attgatgtca tgtatgagga ggaaccttta 600
aaggattatt atacactaat ggatattgcc tacatttata cctggagaag gaatggtcca 660
cttccattga aatacagagt tcgacctact tgtaaaagaa tgaagatcag tcaccagaga 720
gatggactga caaatgctgg agaactggaa agtgactctg ggagtgacaa ggccaacagc 780
ccagcaggag gtattccctc cacctcttct tgtttgccta gccccagtac tccagtgcag 840
tctcctcatc cacagtttcc tcacatttcc agtactatga atggaaccag caacagcccc 900
agcggtaacc accaatcttc ttttgccaat agacctcgaa aatcatcagt aaatgggtca 960
tcagcaactt cttctggttg a 981

<210> 56
<211> 326
<212> PRT
<213> Homo sapiens

<400> 56

Met His Arg Thr Thr Arg Ile Lys Ile Thr Glu Leu Asn Pro His Leu
1               5                   10                  15

Met Cys Val Leu Cys Gly Gly Tyr Phe Ile Asp Ala Thr Thr Ile Ile
            20                  25                  30

Glu Cys Leu His Ser Phe Cys Lys Thr Cys Ile Val Arg Tyr Leu Glu
        35                  40                  45

Thr Ser Lys Tyr Cys Pro Ile Cys Asp Val Gln Val His Lys Thr Arg
    50                  55                  60

Pro Leu Leu Asn Ile Arg Ser Asp Lys Thr Leu Gln Asp Ile Val Tyr
65                  70                  75                  80

Lys Leu Val Pro Gly Leu Phe Lys Asn Glu Met Lys Arg Arg Arg Asp
                85                  90                  95

Phe Tyr Ala Ala His Pro Ser Ala Asp Ala Ala Asn Gly Ser Asn Glu
            100                 105                 110

Asp Arg Gly Glu Val Ala Asp Glu Asp Lys Arg Ile Ile Thr Asp Asp
        115                 120                 125

Glu Ile Ile Ser Leu Ser Ile Glu Phe Phe Asp Gln Asn Arg Leu Asp
    130                 135                 140

Arg Lys Val Asn Lys Asp Lys Glu Lys Ser Lys Glu Glu Val Asn Asp
145                 150                 155                 160

Lys Arg Tyr Leu Arg Cys Pro Ala Ala Met Thr Val Met His Leu Arg
                165                 170                 175

Lys Phe Leu Arg Ser Lys Met Asp Ile Pro Asn Thr Phe Gln Ile Asp
            180                 185                 190

Val Met Tyr Glu Glu Glu Pro Leu Lys Asp Tyr Tyr Thr Leu Met Asp

195                 200                 205

Ile Ala Tyr Ile Tyr Thr Trp Arg Arg Asn Gly Pro Leu Pro Leu Lys
    210                 215                 220

Tyr Arg Val Arg Pro Thr Cys Lys Arg Met Lys Ile Ser His Gln Arg
225                 230                 235                 240

Asp Gly Leu Thr Asn Ala Gly Glu Leu Glu Ser Asp Ser Gly Ser Asp
                245                 250                 255

Lys Ala Asn Ser Pro Ala Gly Gly Ile Pro Ser Thr Ser Ser Cys Leu
            260                 265                 270

Pro Ser Pro Ser Thr Pro Val Gln Ser Pro His Pro Gln Phe Pro His
        275                 280                 285

Ile Ser Ser Thr Met Asn Gly Thr Ser Asn Ser Pro Ser Gly Asn His
    290                 295                 300

Gln Ser Ser Phe Ala Asn Arg Pro Arg Lys Ser Ser Val Asn Gly Ser
305                 310                 315                 320

Ser Ala Thr Ser Ser Gly
                325

<210>  57
<211>  20
<212>  DNA
<213>  Artificial

<220>
<223>  Primer for PEPCK

<400>  57
gcacatccca actctcgatt                                                  20

<210>  58
<211>  20
<212>  DNA
<213>  Artificial

<220>
<223>  Primer for PEPCK

<400>  58
gagggagaac agctgagtgg                                                  20

<210>  59
<211>  20
<212>  DNA
<213>  Artificial

<220>
<223>  Primer for PC

<400>  59
tcctgcagaa tgtgctcaac                                                  20

<210>  60
<211>  20
<212>  DNA
<213>  Artificial

<220>
<223>  Primer for PC

<400>  60
cagcatctgg aaagggatgt                                                    20


<210>  61
<211>  20
<212>  DNA
<213>  Artificial

<220>
<223>  Primer for MDH2

<400>  61
gcagccactt tcacttctcc                                                    20


<210>  62
<211>  20
<212>  DNA
<213>  Artificial

<220>
<223>  Primer for MDH2

<400>  62
cattggtgtt gaacaggtcg                                                    20


<210>  63
<211>  20
<212>  DNA
<213>  Artificial

<220>
<223>  Primer for STAT3

<400>  63
aactcttggg acctggtgtg                                                    20


<210>  64
<211>  20
<212>  DNA
<213>  Artificial

<220>
<223>  Primer for STAT3

<400>  64
taggcgcctc agtcgtatct                                                    20


<210>  65
<211>  20
<212>  DNA
<213>  Artificial

<220>
<223>  Primer for USF1

<400>  65
accccaacgt caagtacgtc                                                    20


<210>  66
<211>  20
<212>  DNA
<213>  Artificial

<220>
<223>  Primer for USF1

<400>  66

acgacctcta atccgtggtg                                                20

<210>  67
<211>  20
<212>  DNA
<213>  Artificial

<220>
<223>  Primer for SREBF1

<400>  67
cggagaagct gcctatcaac                                                20

<210>  68
<211>  20
<212>  DNA
<213>  Artificial

<220>
<223>  Primer for SREBF1

<400>  68
ggtcagtgtg tcctccacct                                                20

<210>  69
<211>  20
<212>  DNA
<213>  Artificial

<220>
<223>  Primer for acetoacetyl-CoA

<400>  69
aaaggcagtc ggctcaacta                                                20

<210>  70
<211>  20
<212>  DNA
<213>  Artificial

<220>
<223>  Primer for acetoacetyl-CoA

<400>  70
gtccagcaca ccattcacac                                                20

<210>  71
<211>  20
<212>  DNA
<213>  Artificial

<220>
<223>  Primer for IPP1

<400>  71
ctcgacaagc aacaggttca                                                20

<210>  72
<211>  20
<212>  DNA
<213>  Artificial

<220>
<223>  Primer for IPP1

<400>  72
ctcagctttc agccgtctct                                                20

<210>  73

<211>  20
<212>  DNA
<213>  Artificial

<220>
<223>  Primer for HMG-CoA

<400>  73
cctggcatca actacccagt                                                    20


<210>  74
<211>  20
<212>  DNA
<213>  Artificial

<220>
<223>  Primer for HMG-CoA

<400>  74
ccagggagat ctcgtagcag                                                    20